(19) Europäisches Patentamt
European Patent Office
Office européen des brevets

(11) **EP 3 553 584 A1**

(12) **EUROPEAN PATENT APPLICATION**
published in accordance with Art. 153(4) EPC

(43) Date of publication:
**16.10.2019 Bulletin 2019/42**

(21) Application number: **17878731.3**

(22) Date of filing: **24.11.2017**

(51) Int Cl.:
*G02B 21/36* (2006.01)  *A61B 3/13* (2006.01)
*A61F 9/007* (2006.01)  *G02B 21/06* (2006.01)

(86) International application number:
**PCT/JP2017/042152**

(87) International publication number:
**WO 2018/105411 (14.06.2018 Gazette 2018/24)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **06.12.2016 JP 2016236708**

(71) Applicant: **Sony Corporation**
**Tokyo 108-0075 (JP)**

(72) Inventor: **ENOKI Junichiro**
**Tokyo 108-0075 (JP)**

(74) Representative: **D Young & Co LLP**
**120 Holborn**
**London EC1N 2DY (GB)**

(54) **IMAGE PROCESSING DEVICE AND METHOD, AND OPERATING MICROSCOPE SYSTEM**

(57)    The present technology relates to an image processing device and a method, and an operating microscope system that enable an operation to be performed more comfortably.

An image processing device includes: an image acquisition unit configured to acquire a coaxial illumination image obtained by performing coaxial illumination on an eye being an operation target, and an oblique illumination image obtained by performing oblique illumination on the eye; and an image processing unit configured to generate an operative field image of the eye on the basis of the coaxial illumination image and the oblique illumination image, or detect a specific region on the basis of at least either one of the coaxial illumination image or the oblique illumination image. The present technology may be applied to a microscope system.

**FIG. 6**

## Description

Technical Field

The present technology relates to an image processing device and method, and an operating microscope system, and particularly to an image processing device and method, and an operating microscope system that enables an operation to be performed more comfortably operation.

Background Art

Conventionally, an ophthalmic microscope has been known. For example, as a technology regarding an ophthalmic microscope, there has been proposed a technology that enables an appropriate tomographic image to be obtained by performing image recognition on an image of an operation target eye that has been acquired during an operation, and deciding a fault surface of the operation target eye on the basis of the recognition result (e.g. refer to Patent Literature 1).

Citation List

Patent Literature

Patent Literature 1: JP 2016-73409A

Disclosure of Invention

Technical Problem

Meanwhile, in an ophthalmic microscope, it is general to observe an operation target eye by simultaneously performing coaxial illumination of emitting illumination light to an observation target eye from an optical axis direction of an observation optical system of the microscope, and oblique illumination of emitting illumination light from a direction different from the optical axis direction of the observation optical system.

This is because transillumination can be obtained by performing the coaxial illumination, so that an anterior capsule and a posterior capsule of a crystalline lens can be observed at good contrast, and shadow contrast can be obtained by performing the oblique illumination, so that an operative field can be stereoscopically observed.

Nevertheless, in the case of observing an operation target eye by performing the coaxial illumination and the oblique illumination, optimum illumination intensities of the coaxial illumination and the oblique illumination vary for each process of an operation, that is to say, vary for each procedure. Thus, for obtaining a good operative field, it is necessary to perform the adjustment of illumination intensities of the coaxial illumination and the oblique illumination for each process during the operation of the eye, which takes a lot of trouble. This prevents an operator or the like from comfortably performing the operation.

The present technology has been devised in view of such a situation, and enables an operation to be performed more comfortably.

Solution to Problem

An image processing device of a first aspect of the present technology includes: an image acquisition unit configured to acquire a coaxial illumination image obtained by performing coaxial illumination on an eye being an operation target, and an oblique illumination image obtained by performing oblique illumination on the eye; and an image processing unit configured to generate an operative field image of the eye on the basis of the coaxial illumination image and the oblique illumination image, or detect a specific region on the basis of at least either one of the coaxial illumination image or the oblique illumination image.

Coaxial illumination light of the coaxial illumination and oblique illumination light of the oblique illumination may be rays of light that have mutually different wavelengths, and the image processing device may further include a control unit configured to control the coaxial illumination and the oblique illumination to be simultaneously performed.

The image acquisition unit may be caused to acquire the coaxial illumination image and the oblique illumination image by optically separating the coaxial illumination light and the oblique illumination light.

The image acquisition unit may be caused to acquire the coaxial illumination image and the oblique illumination image from data obtained by receiving the coaxial illumination light and the oblique illumination light.

The image processing device may further include a control unit configured to control the coaxial illumination and the oblique illumination to be alternately performed. The image acquisition unit may be caused to acquire the coaxial illumination image and the oblique illumination image in a time-shared manner.

The image processing unit may be caused to generate the operative field image by mixing the coaxial illumination image and the oblique illumination image at a predetermined mixing ratio.

The image processing unit may be caused to decide the mixing ratio for each region.

The image processing unit may be caused to detect a region of an operative tool or a region of a specific portion of the eye on the basis of at least either one of the coaxial illumination image or the oblique illumination image, and decide the mixing ratio for each region on the basis of the detection result.

The image processing unit may be caused to decide the mixing ratio on the basis of at least either one of a use application of the operative field image or a process of an operation of the eye.

The image processing unit may be caused to generate the operative field image after performing enhancement processing on at least either one of the coaxial illumination image or the oblique illumination image.

The image processing unit may be caused to decide whether to perform the enhancement processing, on the basis of at least either one of a use application of the operative field image or a process of an operation of the eye, for each of the coaxial illumination image and the oblique illumination image.

The image processing unit may be caused to detect a specular reflection region on the basis of the coaxial illumination image and the oblique illumination image, and generate the operative field image in which a specular reflection component is reduced, on the basis of the detection result.

The image processing unit may be caused to detect a region of a specific portion of the eye as the specific region.

The image processing unit may be caused to compare the coaxial illumination image and the oblique illumination image, and detect a pupil region of the eye.

The image processing device may further include a control unit configured to perform shooting control of the coaxial illumination image and the oblique illumination image on the basis of a detection result of the region of the specific portion.

The control unit may be caused to perform exposure control or control of a shooting position as the shooting control.

The image processing unit may be caused to generate guide information for aiding an operation of the eye, on the basis of a detection result of the region of the specific portion of the eye.

An image processing method of a first aspect of the present technology includes the steps of: acquiring a coaxial illumination image obtained by performing coaxial illumination on an eye being an operation target, and an oblique illumination image obtained by performing oblique illumination on the eye; and generating an operative field image of the eye on the basis of the coaxial illumination image and the oblique illumination image, or detecting a specific region on the basis of at least either one of the coaxial illumination image or the oblique illumination image.

In the first aspect of the present technology, a coaxial illumination image obtained by performing the coaxial illumination to an eye being an operation target and an oblique illumination image obtained by performing the oblique illumination to the eye are acquired, and an operative field image of the eye is generated on the basis of the coaxial illumination image and the oblique illumination image, or a specific region is detected on the basis of at least either one of the coaxial illumination image or the oblique illumination image.

An operating microscope system of a second aspect of the present technology includes: a coaxial illumination light source configured to perform coaxial illumination by emitting coaxial illumination light to an eye being an operation target; an oblique illumination light source configured to perform oblique illumination by emitting oblique illumination light to the eye; an image acquisition unit configured to acquire a coaxial illumination image obtained by performing the coaxial illumination, and an oblique illumination image obtained by performing the oblique illumination; and an image processing unit configured to generate an operative field image of the eye on the basis of the coaxial illumination image and the oblique illumination image, or detect a specific region on the basis of at least either one of the coaxial illumination image or the oblique illumination image.

In the second aspect of the present technology, coaxial illumination is performed by emitting coaxial illumination light to an eye being an operation target, oblique illumination is performed by emitting oblique illumination light to the eye, a coaxial illumination image obtained by performing the coaxial illumination and an oblique illumination image obtained by performing the oblique illumination are acquired, and an operative field image of the eye is generated on the basis of the coaxial illumination image and the oblique illumination image, or a specific region is detected on the basis of at least either one of the coaxial illumination image or the oblique illumination image.

Advantageous Effects of Invention

According to the first aspect and the second aspect of the present technology, an operation can be performed more comfortably.

Note that the advantageous effects described here are not necessarily limitative, and any of the advantageous effects described in the present disclosure may be attained.

Brief Description of Drawings

FIG. 1 is a diagram describing a general ophthalmic microscope.
FIG. 2 is a diagram describing an operative field to be observed by oblique illumination.
FIG. 3 is a diagram describing an operative field to be observed by coaxial illumination.
FIG. 4 is a diagram describing visibility of a shadow in an operative field to be observed by the coaxial illumination.
FIG. 5 is a diagram describing visibility of a shadow in an operative field to be observed by the coaxial illumination and the oblique illumination.
FIG. 6 is a diagram illustrating a configuration example of a microscope system.
FIG. 7 is a diagram illustrating a configuration example of an image acquisition unit.
FIG. 8 is a diagram illustrating a configuration example of a shooting unit.

FIG. 9 is a diagram illustrating another configuration example of a shooting unit.

FIG. 10 is a diagram illustrating another configuration example of a shooting unit.

FIG. 12 is a diagram illustrating another configuration example of a shooting unit.

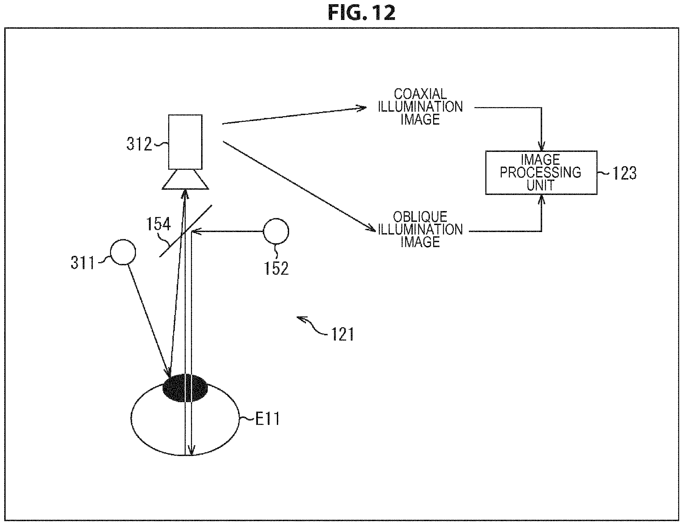

FIG. 12 is a diagram illustrating another configuration example of an image acquisition unit.

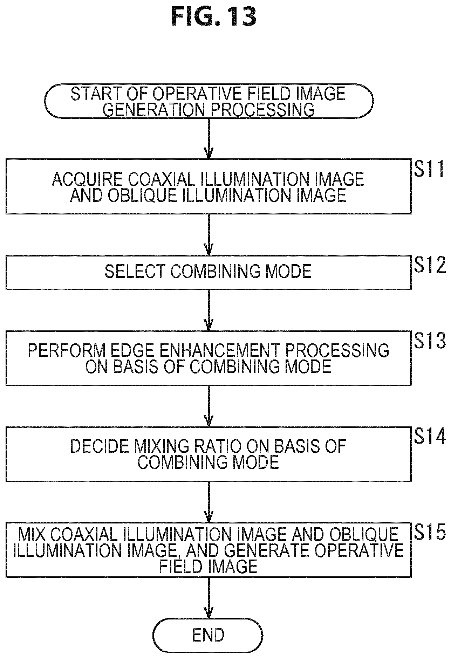

FIG. 13 is a flowchart describing operative field image generation processing.

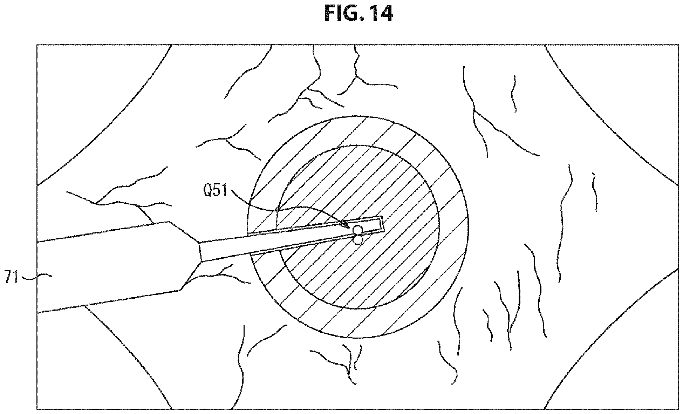

FIG. 14 is a diagram describing a specular reflection region in a coaxial illumination image.

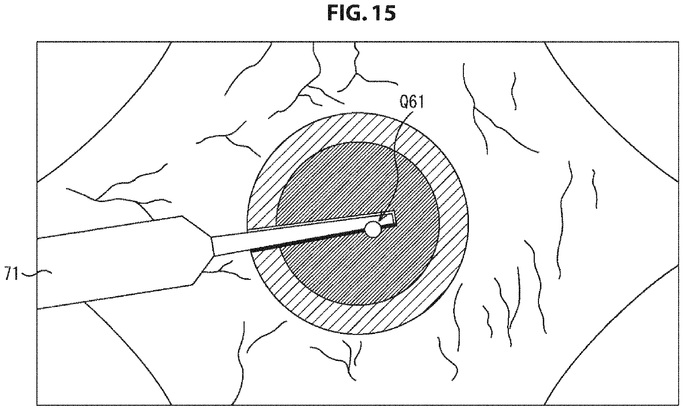

FIG. 15 is a diagram describing a specular reflection region in an oblique illumination image.

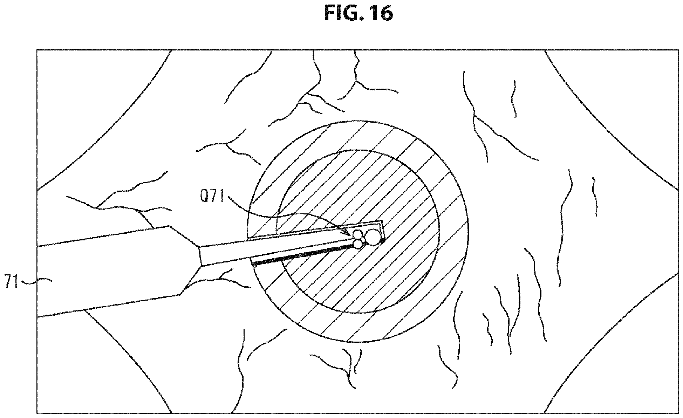

FIG. 16 is a diagram describing a specular reflection region in an operative field image.

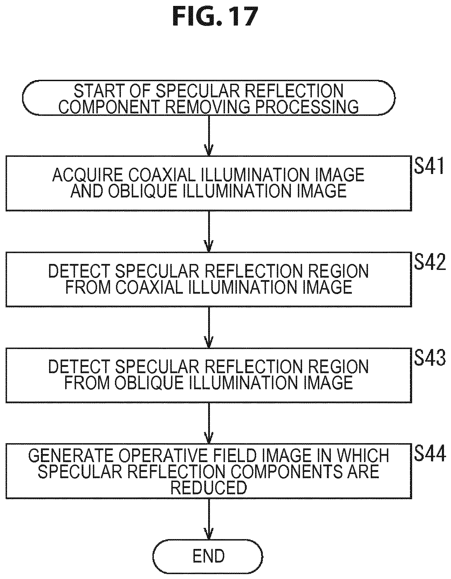

FIG. 17 is a flowchart describing specular reflection component removing processing.

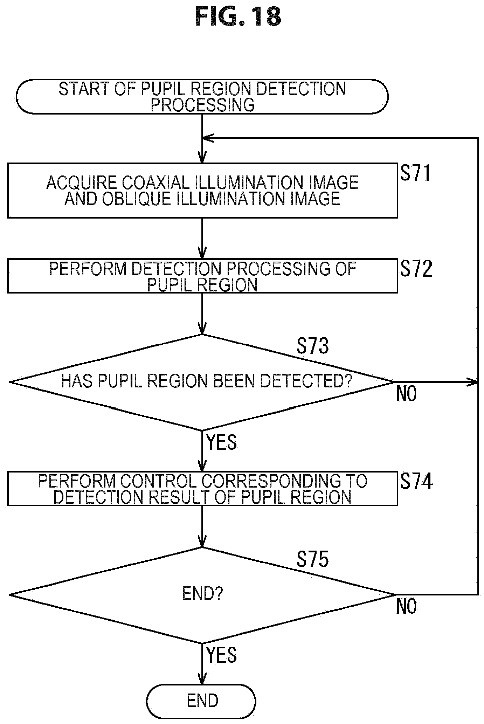

FIG. 18 is a flowchart describing pupil region detection processing.

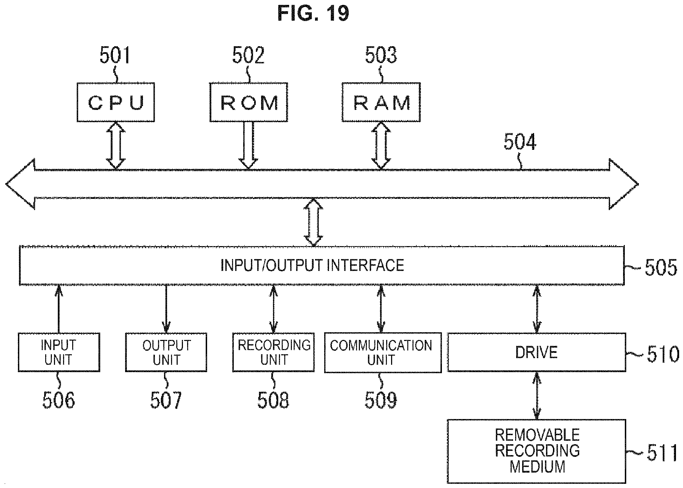

FIG. 19 is a diagram illustrating a configuration example of a computer.

Mode(s) for Carrying Out the Invention

Hereinafter, an embodiment to which the present technology is applied will be described with reference to the drawings.

<First embodiment

<Present technology>

The present technology enables the separate acquisition of a video obtained by coaxial illumination and a video obtained by oblique illumination, by acquiring a coaxial illumination image shot in a state in which an operation target eye is illuminated by coaxial illumination, and an oblique illumination image shot in a state in which an operation target eye is illuminated by oblique illumination. With this configuration, for example, a good operative field can be obtained more easily, and it becomes possible for an operator or the like to perform an operation more comfortably.

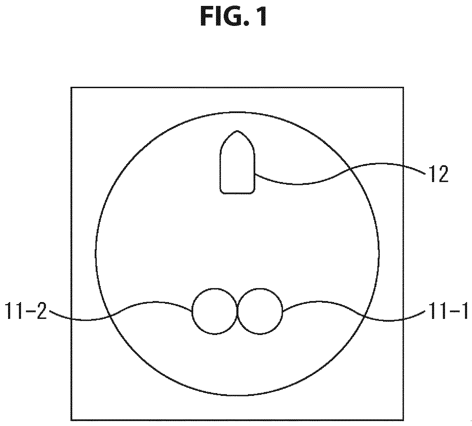

For example, in a general ophthalmic microscope, as illustrated in FIG. 1, there exists coaxial illumination of illuminating a subject from completely the same direction as an optical axis, and oblique illumination of illuminating a subject from a direction shifted by 4 to 6 degrees with respect to the optical axis.

FIG. 1 illustrates an optical system portion of a general ophthalmic microscope. In this example, the microscope is provided with a light source 12 for oblique illumination together with an optical system 11-1 and an optical system 11-2, which are optical systems of an eyepiece lens. Note that, hereinafter, the optical system 11-1 and the optical system 11-2 will also be simply referred to as optical systems 11 in a case where these need not to be specifically distinguished.

The optical system 11-1 and the optical system 11-2 are observation optical systems for observing an operation target eye, and when an eye being a subject is to be observed, illumination light is emitted to the eye via a part of these optical systems 11. Such illumination of a subject from the same direction as an optical axis direction of an observation optical system is called coaxial illumination.

In contrast to this, the ophthalmic microscope is provided with the light source 12 at a position shifted from the optical systems 11, and when an operation target eye is to be observed, illumination light is emitted also by the light source 12 to the eye being a subject. The illumination performed by the light source 12 is illumination from a direction shifted by 4 to 6 degrees from the optical axis direction of the optical systems 11 being observation optical systems, and such illumination is called oblique illumination.

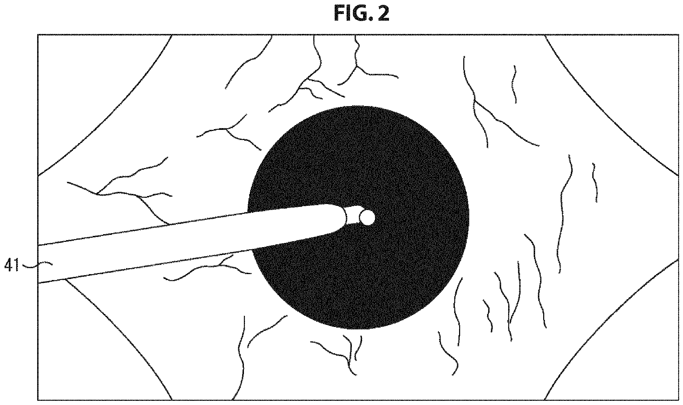

In a case where only the oblique illumination is performed as illumination, transillumination cannot be obtained, that is to say, reflected light from a retina cannot be received. Thus, for example, as illustrated in FIG. 2, in an operative field to be observed in a state in which the oblique illumination is performed, sufficient visibility cannot be obtained.

FIG. 2 illustrates a portion of an operation target eye to be observed in a state in which the oblique illumination is performed.

In this example, in the operative field, an operative tool 41 is inserted into the operation target eye, but in the state, only the oblique illumination has been performed as illumination. Thus, a posterior capsule portion of the eye and the like are dark and sufficient visibility is not obtained.

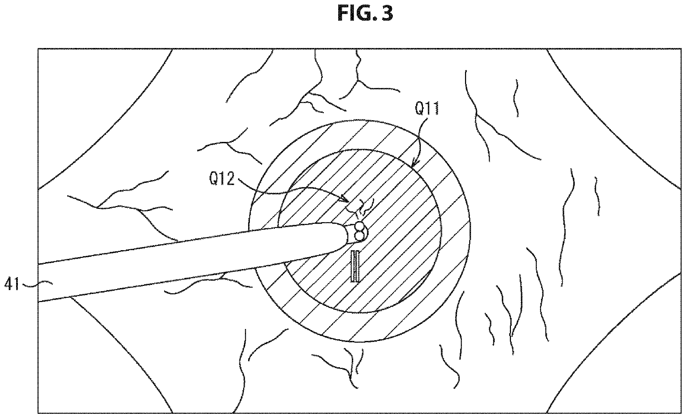

In contrast to this, in the coaxial illumination, by obtaining transillumination, for example, as illustrated in FIG. 3, contrast good for the observation of an anterior capsule and the posterior capsule of a transparent crystal lens can be obtained. Nevertheless, on the other hand, because of being the coaxial illumination, it is impossible to obtain sufficient shadow contrast. Note that, in FIG. 3, a portion corresponding to that in FIG. 2 is denoted with the same reference numeral, and the description thereof will be appropriately omitted.

FIG. 3 illustrates a portion of an operation target eye to be observed in a state in which the coaxial illumination is performed. In this example, in the operative field, the operative tool 41 is inserted into the operation target eye.

In the example of the operative field illustrated in FIG. 3, a portion indicated by an arrow Q11 is a rim portion of an anterior capsule of the eye, and because of the coaxial illumination, each portion of the eye such as a posterior capsule is bright and high visibility is obtained. For example, in a portion indicated by an arrow Q12, even crinkles of a corneal surface that are generated by the insertion of the operative tool 41 can be confirmed.

Nevertheless, because the operative field is illuminated only by the coaxial illumination, a shadow of the operative tool 41 or the like is not observed in the operative field, and sufficient shadow contrast cannot be obtained.

Thus, at the time of an operation of an eye, it is general to combine the coaxial illumination and the oblique illumination, and enable shadow contrast to be obtained by the oblique illumination.

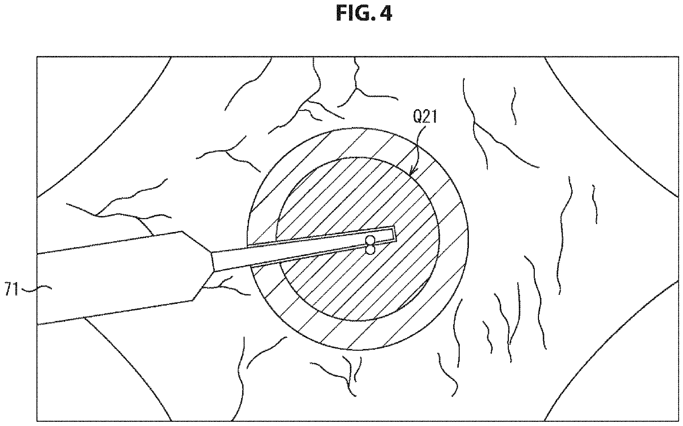

For example, as illustrated in FIG. 4, in an operative field to be observed in a state in which only the coaxial illumination has been performed at the time of phacoemulsification in a cataract operation, while high visibility can be obtained, a shadow of an operative tool 71 or the like is not observed.

In this example, a portion indicated by an arrow Q21 is a rim portion of an anterior capsule of an eye, and because of the coaxial illumination, each portion of the eye such as a posterior capsule is bright and high visibility is obtained. Nevertheless, a shadow of the operative tool 71, a shadow of a groove created by the insertion of the operative tool 71, and the like are not observed, and sufficient shadow contrast is not obtained.

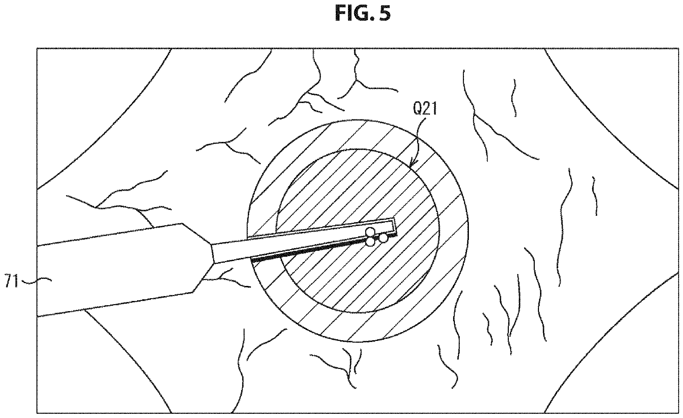

In contrast to this, for example, as illustrated in FIG. 5, in an operative field to be observed in a state in which the coaxial illumination and the oblique illumination have been simultaneously performed at the time of phacoemulsification in a cataract operation, high visibility can be obtained, and sufficient shadow contrast can also be obtained. Note that, in FIG. 5, portions corresponding to those in FIG. 4 are denoted with the same reference numerals, and the description thereof will be appropriately omitted.

In the operative field illustrated in FIG. 5, a groove created by the insertion of the operative tool 71 can be observed, and furthermore, a shadow can be seen in a portion of the groove. In other words, sufficient shadow contrast is obtained. An operator or the like that observes the operative field can sense three-dimensionality from visual information such as the above-described shadow of the groove portion.

Meanwhile, for example, in a cataract operation, there exist a procedure for which contrast is important, such as anterior capsulotomy, and a procedure for which information regarding three-dimensionality is necessary to some extent, as in the excavation of a groove in phacoemulsification.

Thus, the best balance between the illumination intensity of the coaxial illumination and the illumination intensity of the oblique illumination varies for each procedure, and for obtaining a good operative field in an operation, that is to say, a viewing field, a trouble of appropriately adjusting the illumination intensities of the coaxial illumination and the oblique illumination for each procedure, that is to say, for each process of an operation is generated. In other words, it has been difficult to easily obtain a good operative field.

In addition, in a recent ophthalmic surgery, there exists a heads up surgery in which an eye piece of an optical microscope is replaced with a camera, and an operation is performed while viewing a video displayed on a display. In the heads up surgery, the use of image processing is regarded as a merit, and for example, even if a light amount of illumination is decreased, a bright operative field can be obtained by a digital gain.

Nevertheless, also in the heads up surgery, balance control of the illumination intensities of the coaxial illumination and the oblique illumination becomes necessary similarly to visual observation performed using an eyepiece lens, and a trouble is generated.

In addition, because only a video in which illumination light of the coaxial illumination and illumination light of the oblique illumination are mixed can be obtained, it is difficult to increase only the contrast of an anterior capsule or a posterior capsule, or to increase only shadow contrast, for enhancing visibility by image processing, for example.

In view of the foregoing, the present technology enables a good operative field to be obtained more easily, by acquiring a coaxial illumination image shot in a state in which an operation target eye is illuminated by the coaxial illumination, and an oblique illumination image shot in a state in which the operation target eye is illuminated by the oblique illumination. This reduces troubles in an operation, and enables an operator or the like to perform the operation more comfortably.

<Configuration example of microscope system>

Hereinafter, a specific embodiment to which the present technology has been applied will now be described.

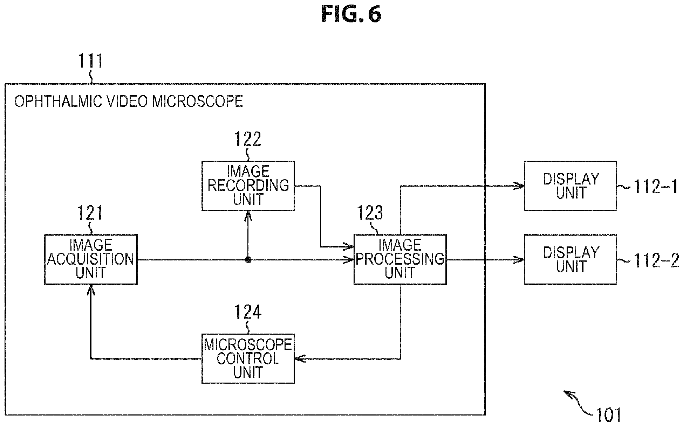

FIG. 6 is a diagram illustrating a configuration example of an embodiment of a microscope system to which the present technology has been applied.

A microscope system 101 illustrated in FIG. 6 includes an ophthalmic video microscope 111, a display unit 112-1, and a display unit 112-2.

By performing shooting and image processing that are interlocked with illumination in the ophthalmic video microscope 111, the microscope system 101 enables image processing that could not be obtained in existing illumination, and accordingly provides an operator with a good operative field and information for supporting an operation.

In the microscope system 101, the two display units 112-1 and 112-2 are connected to the ophthalmic video microscope 111.

In the ophthalmic video microscope 111, shooting is performed while regarding an operation target, that is to say, an eye of a patient to be operated, as a subject, and an image of an operative field in which the eye of the patient is regarded as a subject is generated as an operative field image. In addition, the ophthalmic video microscope 111 supplies the obtained operative field image to the display unit 112-1 or the display unit 112-2 and causes the operative field image to be displayed thereon.

The display unit 112-1 and the display unit 112-2 include liquid crystal displays or the like, for example, and display the operative field image supplied from the ophthalmic video microscope 111. Hereinafter, the display unit 112-1 and the display unit 112-2 will also be simply referred to as display units 112 in a case where these need not to be specifically distinguished.

In addition, these display units 112 may be displays having different use applications. For example, the display unit 112-1 may be provided for presenting an operative field image for observing an operative field, to an operator that performs an operation of an eye, and the display unit 112-2 may be provided for presenting an operative field image to an observer or the like for observing or supervising the operation.

Accordingly, in the ophthalmic video microscope 111, operative field images different for the respective display units 112 can be generated, and the plurality of obtained operative field images can be respectively supplied to the plurality of display units 112.

Note that the number of display units 112 connected to the ophthalmic video microscope 111 is not limited to two, and may be any number. More specifically, one display unit 112 may be connected to the ophthalmic video microscope 111, or three or more display units 112 may be connected thereto.

In addition, the ophthalmic video microscope 111 includes an image acquisition unit 121, an image recording unit 122, an image processing unit 123, and a microscope control unit 124.

In accordance with the control of the microscope control unit 124, the image acquisition unit 121 emits illumination light to an eye serving as an operation target, and acquires an image in which the eye is regarded as a subject. Specifically, the image acquisition unit 121 acquires a coaxial illumination image obtained by performing the coaxial illumination, and an oblique illumination image obtained by performing the oblique illumination, and supplies the images to the image recording unit 122 and the image processing unit 123.

Here, when illumination light emitted to the operation target eye by the coaxial illumination is referred to as coaxial illumination light, a coaxial illumination image is an image obtained by emitting the coaxial illumination light to an eye serving as a subject, receiving the coaxial illumination light reflected on the eye, and photoelectrically converting the light.

In other words, the coaxial illumination image can also be said as an image of an operation target eye that has been shot in a state in which only the coaxial illumination has been performed without performing the oblique illumination.

In addition, when illumination light emitted to the operation target eye by the oblique illumination is referred to as oblique illumination light, an oblique illumination image is an image obtained by emitting the oblique illumination light to an eye serving as a subject, receiving the oblique illumination light reflected on the eye, and photoelectrically converting the light.

In other words, the oblique illumination image can also be said as an image of an operation target eye that has been shot in a state in which only the oblique illumination has been performed without performing the coaxial illumination.

Note that the coaxial illumination image and the oblique illumination image that have been acquired by the image acquisition unit 121 may be supplied to either one of the image recording unit 122 and the image processing unit 123. For example, in a case where the coaxial illumination image and the oblique illumination image are supplied only to the image recording unit 122, the image processing unit 123 reads out the coaxial illumination image and the oblique illumination image from the image recording unit 122, and performs image processing and the like.

Hereinafter, the description will be continued while assuming that a coaxial illumination image and an oblique illumination image are supplied at least to the image processing unit 123.

The image recording unit 122 records the coaxial illumination image and the oblique illumination image that have been supplied from the image acquisition unit 121, and supplies the coaxial illumination image and the oblique illumination image that are recorded thereon, to the image processing unit 123 as necessary.

On the basis of the coaxial illumination image and the oblique illumination image that have been supplied from the image acquisition unit 121 or the image recording unit 122, the image processing unit 123 performs various types of image processing.

For example, the image processing unit 123 generates an operative field image on the basis of a coaxial illumination image and an oblique illumination image, supplies the obtained operative field image to the display unit 112, and causes the operative field image to be displayed thereon. Note that the operative field image obtained by the image processing unit 123 may be supplied to the image recording unit 122 and recorded thereonto.

In addition, for example, on the basis of at least either one of a coaxial illumination image or an oblique illumination image, the image processing unit 123 performs, as image processing, processing of detecting a specific region such as a region of an operative tool or a region of a specific portion of an eye.

Specifically, for example, on the basis of a coaxial illumination image and an oblique illumination image, the image processing unit 123 can identify (detect) a region of a desired portion of an operation target eye on an operative field image, and furthermore, control the display unit 112 on the basis of the identification result to display guide information for aiding an operation, or the like, with being superimposed onto the operative field image.

Furthermore, for example, the image processing unit 123 supplies, to the microscope control unit 124, a result of image processing that uses a coaxial illumination image and an oblique illumination image, that is to say, an identification result (detection result) of a region of a specific portion on the operative field image, for example.

On the basis of the result of image processing or the like that has been supplied from the image processing unit 123, the microscope control unit 124 controls the image acquisition unit 121.

For example, on the basis of the result of image processing or the like, the microscope control unit 124 controls illumination such as the coaxial illumination or the oblique illumination that is performed by the image acquisition unit 121, and controls acquisition (shooting) of a coaxial illumination image or an oblique illumination image that is performed by the image acquisition unit 121.

Specifically, for example, a timing at which illumination is to be performed, the adjustment of an illumination intensity, and the like can be controlled as illumination control. In addition, for example, the control of shooting timings of a coaxial illumination image and an oblique illumination image, exposure control (light exposure control), the control of a shooting position, and the like can be performed as the control of the acquisition of images.

<Configuration example 1 of image acquisition unit >

Subsequently, a specific configuration example of the image acquisition unit 121 illustrated in FIG. 6 will be described.

For example, a configuration of the ophthalmic video microscope 111 can be a configuration of performing shooting and image processing that are interlocked with illumination performed using rays of illumination light that have mutually different wavelengths.

In such a case, the shooting of a coaxial illumination image and an oblique illumination image can be simultaneously performed using coaxial illumination light and oblique illumination light as rays of light that have mutually different wavelengths.

Here, for example, the coaxial illumination light is assumed to be visible light, that is to say, more specifically, light with a visible light component from which an infrared light component has been removed (cut), and the oblique illumination light is assumed to be infrared light.

If the coaxial illumination light and the oblique illumination light are assumed to be rays of light that have mutually different wavelength bands in this manner, the rays of light can be dispersed using a prism or the like, for example, and states of an eye at completely the same time can be acquired as a coaxial illumination image and an oblique illumination image. In other words, a coaxial illumination image and an oblique illumination image at the same time can be separately acquired.

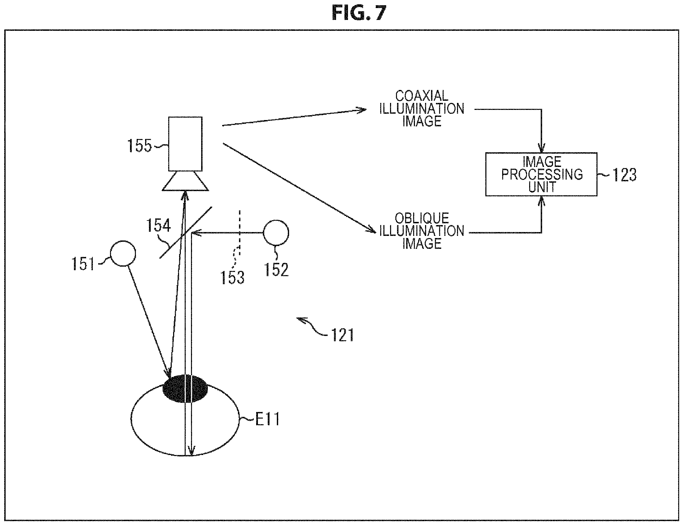

Specifically, in a case where the coaxial illumination light and the oblique illumination light are assumed to be rays of light that have mutually different wavelengths, the image acquisition unit 121 can have a configuration illustrated in FIG. 7, for example. Note that, in FIG. 7, portions corresponding to those in FIG. 6 are denoted with the same reference numerals, and the description thereof will be appropriately omitted.

In the example illustrated in FIG. 7, the image acquisition unit 121 includes an infrared light source 151, a visible light source 152, an infrared cut film 153, a half mirror 154, and a shooting unit 155.

The infrared light source 151 is an oblique illumination light source that emits infrared light to an eye E11 being an operation target, as oblique illumination light, and performs the oblique illumination. Note that an optical system that guides the oblique illumination light to the eye E11 may be provided between the infrared light source 151 and the eye E11 in the image acquisition unit 121.

The oblique illumination light emitted to the eye E11 is reflected on an anterior capsule, a posterior capsule, or the like of the eye E11, for example, and enters the shooting unit 155 via the half mirror 154.

The visible light source 152 is a coaxial illumination light source that emits visible light to the eye E11 being an operation target, as coaxial illumination light, and performs the coaxial illumination. More specifically, the coaxial illumination light output from the visible light source 152 enters the half mirror 154 via the infrared cut film 153 and is reflected on the half mirror 154, and enters the eye E11.

At this time, more specifically, an infrared light component included in the coaxial illumination light is removed (cut) by the infrared cut film 153. In other words, only a visible light component of the coaxial illumination light goes through the infrared cut film 153.

In addition, more specifically, a coaxial illumination optical system that includes the half mirror 154 and another optical element, and guides the coaxial illumination light to the eye E11 is provided between the visible light source 152 and the eye E11 in the image acquisition unit 121. A part of the coaxial illumination optical system is an optical system partially shared with an observation optical system (shooting optical system) for observing the eye E11 by the shooting unit 155, and the coaxial illumination is thereby implemented.

The coaxial illumination light emitted to the eye E11 is reflected on the anterior capsule, the posterior capsule, a retina, or the like of the eye E11, for example, and enters the shooting unit 155 via the half mirror 154.

The infrared cut film 153 is a filter that cuts an infrared light component and lets through only a visible light component, and causes only a visible light component of the coaxial illumination light that has entered from the visible light source 152, to go through and enter the half mirror 154.

The half mirror 154 reflects a half of the coaxial illumination light that has entered from the visible light source 152 via the infrared cut film 153 and causes the light to enter the eye E11, and lets through the remaining half of the coaxial illumination light that has entered.

In a similar manner, the half mirror 154 lets through a half of the oblique illumination light and coaxial illumination light that have entered from the eye E11 and causes the light to enter the shooting unit 155, and reflects the remaining half of the oblique illumination light and coaxial illumination light.

The shooting unit 155 acquires an oblique illumination image and a coaxial illumination image by receiving and photoelectrically converting the oblique illumination light and the coaxial illumination light that have entered from the eye E11 via the half mirror 154. In other words, the shooting unit 155 functions as an infrared camera that shoots an oblique illumination image, and also functions as a visible light camera that shoots a coaxial illumination image.

The shooting unit 155 outputs the coaxial illumination image and the oblique illumination image that have been obtained by the shooting, to the image processing unit 123.

<Configuration example 1 of shooting unit>

Here, a more detailed configuration example of the shooting unit 155 illustrated in FIG. 7 will be described.

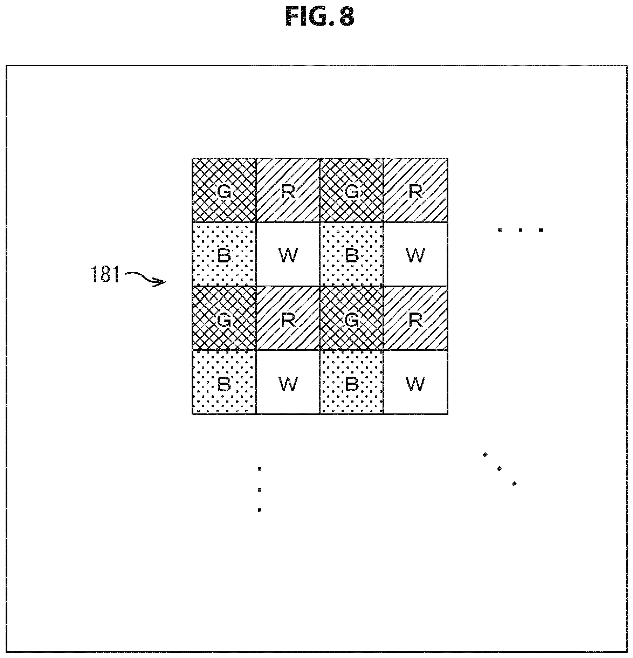

For example, the shooting unit 155 can be a camera including an image sensor having a pixel array as illustrated in FIG. 8, that is to say, an image sensor 181.

The image sensor 181 illustrated in FIG. 8 is a W pixel introduction sensor in which a color filter is provided for each pixel on the surface of a pixel light receiving unit. Here, one square represents one pixel, and letters in these pixels represent color filters provided for these pixels.

More specifically, a pixel marked with a letter "R" is a pixel provided with a color filter for letting through light with an R component, that is to say, red light, and is a pixel that receives light with an R component (hereinafter, will also be referred to as an R pixel).

In addition, a pixel marked with a letter "G" is a pixel provided with a color filter for letting through light with a G component, that is to say, green light, and is a pixel that receives light with a G component (hereinafter, will also be referred to as a G pixel). A pixel marked with a letter "B" is a pixel provided with a color filter for letting through light with a B component, that is to say, blue light, and is a pixel that receives light with a B component (hereinafter, will also be referred to as a B pixel).

Furthermore, a pixel marked with a letter "W" is a pixel provided with a color filter for letting through light with a W component, that is to say, light with all wavelength bands (white light), and is a pixel that receives light with a W component (hereinafter, will also be referred to as a W pixel).

In FIG. 8, in the image sensor 181, the array of pixels including R pixels, G pixels, B pixels, and W pixels is assumed to be an array in which a part of a bayer array, more specifically, a part of G pixels in the bayer array is replaced with W pixels.

At the time of image acquisition, because the coaxial illumination light being visible light and the oblique illumination light being infrared light simultaneously enter the image sensor 181, the image sensor 181 shoots an image by receiving and photoelectrically converting light that has entered a pixel via a color filter.

By employing the above-described configuration, from data with three primary colors that has been obtained by the photoelectric conversion in R pixels, G pixels, and B pixels, and data obtained by the photoelectric conversion in W pixels, an image of visible light which includes RGB-color components and from which an infrared light component has been removed, and an image of infrared light which only includes an infrared light component can be obtained. Then, the image of visible light that has been obtained in this manner is regarded as a coaxial illumination image, and the image of infrared light is regarded as an oblique illumination image.

If the camera including the image sensor 181 illustrated in FIG. 8 as described above is used as the shooting unit 155, a coaxial illumination image and an oblique illumination image can be separately acquired by a single sensor, that is to say, the image sensor 181.

<Configuration example 2 of shooting unit>

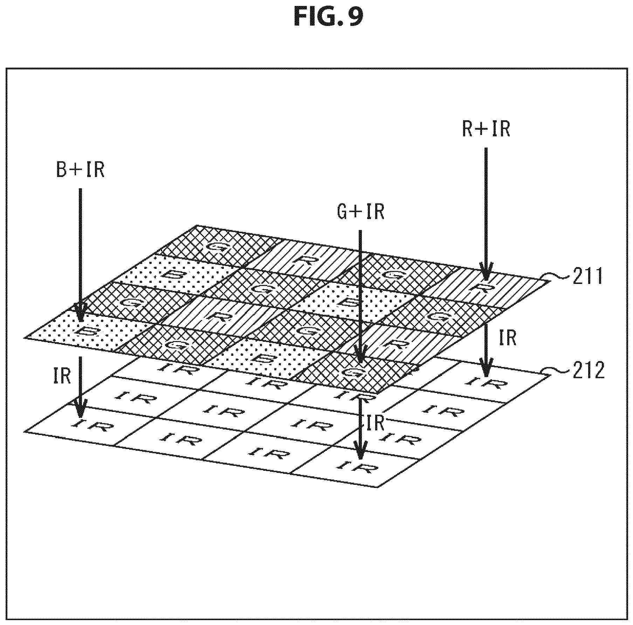

In addition, the shooting unit 155 may also be a camera including a two-layer structured sensor portion as illustrated in FIG. 9, for example.

In the example illustrated in FIG. 9, the shooting unit 155 includes an image sensor 211 and an image sensor 212, which are image sensors disposed with being overlapped with each other. In particular, here, the image sensor 211 is disposed on the half mirror 154 side in the shooting unit 155, that is to say, an incidence side of light.

The upper-layer image sensor 211 is an image sensor in which a color filter is provided for each pixel on the surface of a pixel light receiving unit. Here, one square represents one pixel, and letters in these pixels represent color filters provided for these pixels.

More specifically, a pixel marked with a letter "R" indicates an R pixel provided with a color filter for letting through light with an R component, and a pixel marked with a letter "G" indicates a G pixel provided with a color filter for letting through light with a G component. In addition, a pixel marked with a letter "B" indicates a B pixel provided with a color filter for letting through light with a B component.

In the image sensor 211, these R pixels, G pixels, and B pixels are arranged in the bayer array.

In addition, the lower-layer image sensor 212 disposed on the downside of the image sensor 211 in the drawing is an image sensor including pixels having the same size as each pixel of the image sensor 211. Note that, in the image sensor 212, one square represents one pixel, and a letter "IR" in these pixels indicates an infrared light component being a component of light to be received by the pixels.

When visible light and infrared light enter the pixels of the image sensor 211, a part of the visible light and the infrared light is received by pixels of the image sensor 211, and a remaining part of the rays of light that have entered goes through pixels of the image sensor 211 and enter pixels of the image sensor 212.

In particular, components that go through the pixels of the image sensor 211 are mainly infrared light components, and in the pixels of the image sensor 212, the infrared light components are received.

Because coaxial illumination light being visible light and oblique illumination light being infrared light simultaneously enter the image sensor 211 at the time of image acquisition, the image sensor 211 shoots an image by receiving and photoelectrically converting light that has entered. At the same time, the image sensor 212 also shoots an image by receiving and photoelectrically converting light that has gone through the image sensor 211.

By employing the above-described configuration, from data obtained by the photoelectric conversion in each pixel of the image sensor 211, and data obtained by the photoelectric conversion in each pixel of the image sensor 212, an image of visible light which includes RGB-color components and from which an infrared light component has been removed can be obtained. In addition, for example, from data obtained by the photoelectric conversion in each pixel of the image sensor 212, an image of infrared light which only includes an infrared light component can be obtained. Then, the image of visible light that has been obtained in this manner is regarded as a coaxial illumination image, and the image of infrared light is regarded as an oblique illumination image.

<Configuration example 3 of shooting unit>

Furthermore, the shooting unit 155 may be a camera having a configuration of optically separating the coaxial illumination light and the oblique illumination light before receiving light, that is to say, before shooting. More specifically, for example, infrared light and visible light may be separated by a prism obtained by combining a dichroic film and reflection.

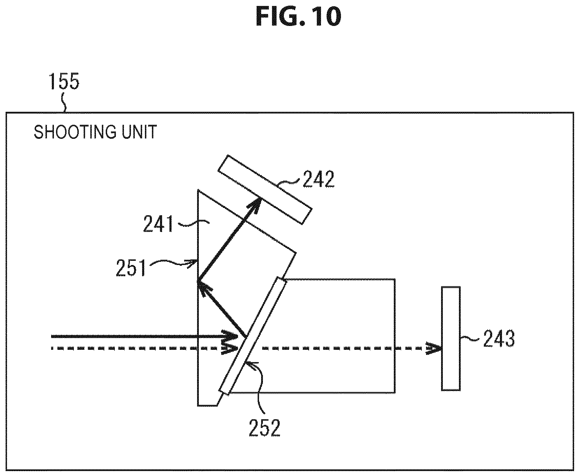

In such a case, the shooting unit 155 has a configuration as illustrated in FIG. 10, for example.

The shooting unit 155 illustrated in FIG. 10 includes a prism 241, an image sensor 242, and an image sensor 243.

In this example, the prism 241 is disposed in such a manner that an incidence plane 251 of the prism 241 is located on the half mirror 154 side, that is to say, the incidence side of light, and a dichroic film 252 is provided in a portion on a plane of the prism 241 that faces the incidence plane 251.

For example, the dichroic film 252 reflects light having a wavelength longer than a wavelength of infrared light, among rays of light that have entered, and lets through light having a wavelength shorter than the wavelength of infrared light, among the rays of light that have entered.

Accordingly, in the shooting unit 155, in the prism 241, the coaxial illumination light being visible light and the oblique illumination light being infrared light can be optically separated.

More specifically, when the coaxial illumination light and the oblique illumination light go through the incidence plane 251 of the prism 241, and these rays of light enter the dichroic film 252, the oblique illumination light being infrared light is reflected on the dichroic film 252, and the coaxial illumination light being visible light goes through the dichroic film 252.

Then, the oblique illumination light reflected on the dichroic film 252 is further reflected on the incidence plane 251, and enters the image sensor 242 being an image sensor. The image sensor 242 acquires an oblique illumination image only including a component of the oblique illumination light, by receiving and photoelectrically converting the oblique illumination light that has entered. In other words, an oblique illumination image is shot by the image sensor 242.

In contrast to this, the coaxial illumination light that has gone through the dichroic film 252 enters the image sensor 243 being an image sensor. The image sensor 243 acquires a coaxial illumination image only including a component of the coaxial illumination light, by receiving and photoelectrically converting the coaxial illumination light that has entered. In other words, a coaxial illumination image is shot by the image sensor 243.

Note that, in this configuration, infrared light is reflected on the dichroic film 252, but visible light may be reflected on the dichroic film 252.

In such a case, the coaxial illumination light that has entered the prism 241 is reflected on the dichroic film 252, and then, further reflected on the incidence plane 251, and enters the image sensor 242. In addition, the oblique illumination light that has entered the prism 241 goes through the dichroic film 252, and enters the image sensor 243. Accordingly, in this case, a coaxial illumination image is shot by the image sensor 242 and an oblique illumination image is shot by the image sensor 243.

<Configuration example 4 of shooting unit>

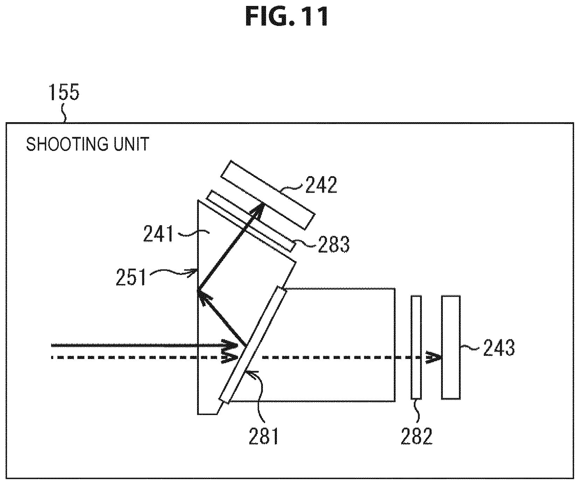

Furthermore, in the case of optically separating the coaxial illumination light and the oblique illumination light in the shooting unit 155 before shooting, the shooting unit 155 can also have a configuration illustrated in FIG. 11. Note that, in FIG. 11, portions corresponding to those in FIG. 10 are denoted with the same reference numerals, and the description thereof will be appropriately omitted.

The shooting unit 155 illustrated in FIG. 11 includes the prism 241, a half mirror 281, a filter 282, a filter 283, the image sensor 242, and the image sensor 243.

In the configuration of the shooting unit 155 illustrated in FIG. 11, the half mirror 281 is disposed in place of the dichroic film 252 of the shooting unit 155 illustrated in FIG. 10, and furthermore, the filter 282 and the filter 283 are provided.

In particular, in the shooting unit 155 illustrated in FIG. 11, the filter 282 is disposed between the half mirror 281 and the image sensor 243, and the filter 283 is disposed between the incidence plane 251 and the image sensor 242.

Here, the filter 282 includes a bandpass filter that lets through only light having a specific wavelength band such as, for example, a wavelength band of infrared light, or the like. In addition, the filter 283 is assumed to be an infrared cut filter or the like, for example, removes (cuts) an infrared light component of the light that has entered, and lets through the other components.

Accordingly, in this example, when the coaxial illumination light and the oblique illumination light go through the incidence plane 251 of the prism 241, and these rays of light enter the half mirror 281, a half of the coaxial illumination light and the oblique illumination light goes through the half mirror 281 and enters the filter 282. On the other hand, a remaining half of the coaxial illumination light and the oblique illumination light that have entered the half mirror 281 is reflected on the half mirror 281, and further reflected also on the incidence plane 251, and enters the filter 283.

Then, the coaxial illumination light out of the coaxial illumination light and the oblique illumination light that have entered the filter 282 is cut by the fill 282, and only the oblique illumination light goes through the filter 282 and enters the image sensor 243. In addition, the oblique illumination light out of the coaxial illumination light and the oblique illumination light that have entered the filter 283 is cut by the fill 283, and only the coaxial illumination light goes through the filter 283 and enters the image sensor 242.

An oblique illumination image is thereby shot by the image sensor 243 and a coaxial illumination image is shot by the image sensor 242.

Note that, here, the description has been given of an example in which an oblique illumination image is shot by the image sensor 243, but an oblique illumination image may be shot by the image sensor 242 and a coaxial illumination image may be shot by the image sensor 243. In such a case, a bandpass filter that lets through a wavelength band of visible light is used as the filter 282, and a visible light cut filter is used as the filter 283.

<Configuration example 2 of image acquisition unit>

In addition, the configuration of the image acquisition unit 121 illustrated in FIG. 6 is not limited to the configuration illustrated in FIG. 7, and may be any other configurations as long as a coaxial illumination image and an oblique illumination image can be acquired.

For example, the configuration of the ophthalmic video microscope 111 can be a configuration of performing shooting and image processing that are interlocked with illumination performed by an illumination light source that can be turned on and off at high speed.

In such a case, using illumination light sources that can turn on and off light emission at high speed, as light sources of the coaxial illumination light or the oblique illumination light, the coaxial illumination light and the oblique illumination light can be emitted to an operation target eye at different timings, and a coaxial illumination image and an oblique illumination image can be acquired in a time-shared manner.

In this case, by performing shooting at a high frame rate using one camera in synchronization with the on/off of the light sources, a coaxial illumination image and an oblique illumination image can be separately acquired in a time-shared manner. At this time, the illumination images can be acquired by regarding an even-numbered frame of a video (moving image) obtained by the shooting, as the oblique illumination image, and regarding an odd-numbered frame as the coaxial illumination image, for example.

In a case where a coaxial illumination image and an oblique illumination image are acquired in a time-shared manner in this manner, unlike the case where the image acquisition unit 121 has the configuration illustrated in FIG. 7, strictly speaking, the coaxial illumination image and the oblique illumination image that are to be acquired do not become videos of completely the same time. Nevertheless, by performing shooting at a sufficiently-high frame rate, the influence of a difference in shooting time between the coaxial illumination image and the oblique illumination image can be made sufficiently small to such a level that an operator or the like does not visually care, for example. In addition, in this case, both images of the coaxial illumination image and the oblique illumination image can be images of visible light.

In a case where a coaxial illumination image and an oblique illumination image are acquired in a time-shared manner, the image acquisition unit 121 has a configuration as illustrated in FIG. 12, for example. Note that, in FIG. 12, portions corresponding to those in FIG. 7 are denoted with the same reference numerals, and the description thereof will be appropriately omitted.

In the example illustrated in FIG. 12, the image acquisition unit 121 includes a visible light source 311, the visible light source 152, the half mirror 154, and a shooting unit 312.

In this example, the visible light source 311 emits visible light as oblique illumination light, and the visible light source 152 emits visible light as coaxial illumination light.

In the image acquisition unit 121 illustrated in FIG. 12, the visible light source 311 is provided in place of the infrared light source 151 illustrated in FIG. 7. The visible light source 311 is an oblique illumination light source that emits visible light to the eye E11 being an operation target, as the oblique illumination light, and performs the oblique illumination. The oblique illumination light emitted to the eye E11 is reflected on an anterior capsule, a posterior capsule, or the like of the eye E11, for example, and enters the shooting unit 312 via the half mirror 154.

In addition, the shooting unit 312 is a camera or the like that can shoot a moving image of visible light at a high frame rate, and extracts a coaxial illumination image and an oblique illumination image from the shot moving image, and supplies the extracted images to the image processing unit 123.

In shooting the coaxial illumination image and the oblique illumination image, the microscope control unit 124 alternately turns on/off the visible light source 311 and the visible light source 152 at high speed.

More specifically, the microscope control unit 124 controls the visible light source 311 and the visible light source 152 so as to alternately switch a state at high speed between an oblique illumination output state in which the oblique illumination light is output from the visible light source 311 and the coaxial illumination light is not output from the visible light source 152, and a coaxial illumination output state in which the oblique illumination light is not output from the visible light source 311 and the coaxial illumination light is output from the visible light source 152.

At the same time, the microscope control unit 124 causes the shooting unit 312 to shoot a moving image in synchronization with the switching between the oblique illumination output state and the coaxial illumination output state. More specifically, the shooting of a moving image that is performed by the shooting unit 312 is controlled in such a manner that a period of the oblique illumination output state becomes a period corresponding to one frame of the moving image, and a period of the coaxial illumination output state becomes a period corresponding to another one frame.

With this configuration, in the moving image obtained by the shooting unit 312, for example, an odd-numbered frame becomes a coaxial illumination image and an even-numbered frame becomes an oblique illumination image. By separating the odd-numbered frame and the even-numbered frame of the shot moving image, the shooting unit 312 acquires the coaxial illumination image and the oblique illumination image, and outputs the images to the image processing unit 123 provided on a subsequent stage.

Note that, here, the description will be given of an example in which the coaxial illumination image and the oblique illumination image are alternately acquired by one frame, but coaxial illumination images corresponding to two frames may be acquired, and then, an oblique illumination image corresponding only to one frame may be acquired, for example.

In addition, here, the description has been given of an example in which the coaxial illumination light and the oblique illumination light are rays of light that have the same wavelength, but the coaxial illumination light and the oblique illumination light may be rays of light that have mutually different wavelengths.

As described above, if the image acquisition unit 121 has the configuration illustrated in FIG. 7, or has the configuration illustrated in FIG. 12, the coaxial illumination image and the oblique illumination image can be separately acquired.

In a case where image processing is performed in real time after the acquisition of a coaxial illumination image and an oblique illumination image, the obtained coaxial illumination image and the oblique illumination image are supplied to the image processing unit 123, and various types of image processing are performed by the image processing unit 123. For example, processing of generating an operative field image, and the like are performed as image processing.

Then, an operative field image or the like that has been obtained as a processing result of the image processing unit 123 is supplied to the display unit 112 and displayed thereon, or an operation of the image acquisition unit 121 is controlled by the microscope control unit 124 on the basis of a processing result of the image processing unit 123.

On the other hand, for example, in a case where image processing is not performed in real time, a coaxial illumination image and an oblique illumination image are individually supplied to the image recording unit 122 are recorded thereon. Then, for example, when an operation or the like is to be reviewed, image processing is performed by the image processing unit 123 on the basis of the coaxial illumination image and the oblique illumination image that are recorded on the image recording unit 122, and an operative field image or the like that has been accordingly obtained is supplied to the display unit 112 and displayed thereon.

By separately acquiring the coaxial illumination image and the oblique illumination image in this manner, in the image processing unit 123, from the coaxial illumination image and the oblique illumination image, an operative field image or the like that has high visibility can be generated for each procedure by image processing. With this configuration, a good operative field can be obtained more easily, and as a result, an operator or the like can perform an operation more comfortably.

In addition, for example, a specific portion of an operation target eye can be detected by image processing from the coaxial illumination image and the oblique illumination image. By using the detection result, guide information that serves as a guide useful for the operation of the eye can be presented, shooting control such as exposure control suitable for the specific portion of the eye can be performed, and an operator or the like can perform the operation more comfortably.

<Description of operative field image generation processing>

Hereinafter, an example of functions to be implemented by the microscope system 101 will now be specifically described.

First of all, processing of generating an operative field image from a coaxial illumination image and an oblique illumination image in the microscope system 101 will be described.

In the microscope system 101, a coaxial illumination image in which good contrast is obtained by the coaxial illumination for an anterior capsule and a posterior capsule, and an oblique illumination image in which sufficient shadow contrast is obtained by the oblique illumination are mixed (combined) at a free mixing ratio for each procedure or for each observer, and an operative field image having high visibility, that is to say, a good operative field can be generated.

Specifically, when the coaxial illumination is performed, contrast of an anterior capsule, a posterior capsule, and a cornea, which are transparent bodies in the operative field, is increased by transillumination, and the stability or the like of an incision rim and the posterior capsule can be checked. On the other hand, when the oblique illumination is performed, by shadow contrast obtained by the oblique illumination, information regarding three-dimensionality such as a shadow of an operative tool in the operative field and the depth of a groove in phacoemulsification can be obtained.

Nevertheless, both of the contrast of these transparent bodies and the shadow contrast of the operative tool or the like are information obtained when these members look dark as compared with the surrounding. Thus, depending on a case, for example, the shadow contrast deteriorates the visibility of the posterior capsule.

Thus, typically, the coaxial illumination and the oblique illumination have been simultaneously performed, and the adjustment of balance between illumination intensities in these types of illumination has been performed.

In contrast to this, in the microscope system 101, because a coaxial illumination image and an oblique illumination image are individually acquired, the coaxial illumination image and the oblique illumination image can be mixed by image processing at an arbitrary ratio, that is to say, at an arbitrary mixing ratio, and the mixed image can be used as an operative field image.

In other words, in the microscope system 101, the adjustment of balance between the intensity of the coaxial illumination and the intensity of the oblique illumination can be performed also by image processing.

Specifically, for example, in a case where the image acquisition unit 121 has the configuration illustrated in FIG. 12, both images of the coaxial illumination image and the oblique illumination image can be visible light images.

Accordingly, in this case, an operative field image can be obtained by mixing, at an arbitrary mixing ratio, pixel values of pixels of the coaxial illumination image and the oblique illumination image that are in the same positional relationship, that is to say, by weighted-adding the pixel values. More specifically, when a pixel of interest of an operative field image to be obtained is called a target pixel, by weighted-adding a pixel value of a pixel of the coaxial illumination image that is in the same positional relationship as the target pixel, and a pixel value of a pixel of the oblique illumination image that is in the same positional relationship as the target pixel, using a weight defined by the mixing ratio, a pixel value of the target pixel can be obtained.

In contrast to this, for example, in a case where the image acquisition unit 121 has the configuration illustrated in in FIG. 7, while a coaxial illumination image becomes a visible light image, an oblique illumination image becomes an infrared light image, that is to say, a monochrome image.

Nevertheless, also in such a case where, by mixing an image with luminance forming the coaxial illumination image and the oblique illumination image, a colored operative field image, that is to say, a color operative field image can be obtained.

More specifically, first of all, image conversion processing is performed as necessary on a color coaxial illumination image being a visible light image, and the resultant image is regarded as a coaxial illumination image including a Y image, Cb image, and a Cr image. Here, the Y image is a luminance image including a Y component (luminance component), the Cb image is a color difference image including a Cb component (blue color difference component), and the Cr image is a color difference image including a Cr component (red color difference component).

After that, pixel values of pixels in the same positional relationship of the oblique illumination image being a monochrome image and the Y image forming the coaxial illumination image are mixed at an arbitrary mixing ratio, and the resultant image is regarded as a mixed luminance image. Then, a color image including the mixed luminance image, the Cb image, and the Cr image that have been obtained in this manner is regarded as an operative field image.

Note that, hereinafter, processing of generating an operative field image from a coaxial illumination image and an oblique illumination image being visible light images, and processing of generating an operative field image from a coaxial illumination image being a visible light image and an oblique illumination image being an infrared light image will also be referred to as mix processing without specifically making a distinction. In other words, also in the case of generating an operative field image from an oblique illumination image being a monochrome image and a coaxial illumination image being a visible light image, the processing will also be simply referred to as mixing the oblique illumination image and the coaxial illumination image.

In addition, in the case of generating an operative field image by performing the mix processing as image processing, the coaxial illumination image and the oblique illumination image can also be mixed at a mixing ratio different for each region.

For example, it becomes important to recognize a three-dimensional position of an operative tool being manipulated by an operator. Thus, an operative field image having easy-to-recognize three-dimensionality may be enabled to be obtained by increasing a contributing rate of the oblique illumination image with respect to the operative field image, that is to say, a mixing ratio of the oblique illumination image in the mix processing as for a region around the operative tool.

At this time, as an identification method of a region of the operative tool in the coaxial illumination image and the oblique illumination image, for example, the region can be identified by performing object recognition (image recognition) or the like on the coaxial illumination image and the oblique illumination image.

Aside from this, the region of the operative tool may be identified by adding a marker having a specific color or shape to the operative tool, and detecting the marker from the coaxial illumination image and the oblique illumination image, or an observer or the like may designate the region of the operative tool by an input.

In addition, for example, as described later with reference to a flowchart in FIG. 18, a region of a specific portion of an eye may be identified (detected) by comparing the coaxial illumination image and the oblique illumination image and identifying a pupil region, and a mixing ratio of each region may be decided in accordance with the identification result.

In the case of deciding a mixing ratio for each region in this manner, it is only required that a region of an operative tool or a specific portion of an eye is detected by using at least either one of a coaxial illumination image or an oblique illumination image, and a mixing ratio of each region is decided on the basis of the detection result.

Furthermore, an operative field image may be generated after performing edge enhancement processing as image processing on at least either one of the coaxial illumination image or the oblique illumination image.

For example, by performing the edge enhancement processing on a coaxial illumination image having no shadow, and then, mixing the coaxial illumination image having been subjected to the edge enhancement processing, and an oblique illumination image not having been subjected to the edge enhancement processing, it becomes possible to obtain an operative field image having enhanced visibility of a transparent body, without enhancing an edge component of a shadow obtained by the oblique illumination. In this manner, by appropriately performing the edge enhancement processing on the coaxial illumination image and the oblique illumination image in accordance with a use application, an operative field image having higher visibility can be obtained.

Note that, here, the description will be given of a case where the edge enhancement processing is performed as an example of image processing, but aside from this, for example, other types of display enhancement processing such as contrast enhancement processing may be performed.

A mixing ratio, the presence or absence of the edge enhancement processing, and the like of each region or the entire image in the mix processing that have been described above can be changed in accordance with a presentation destination such as an operator or an observer to which an operative field image is to be presented, that is to say, a use application of the operative field image. In other words, depending on the display unit 112 to which an operative field image is to be supplied, the mix processing can be performed at a different mixing ratio or whether or not to perform the edge enhancement processing can be selectively switched.

For example, conceivable cases include the case of generating an operative field image to be presented to an observer who does not manipulate an operative tool, such as a supervisory doctor or an assistant, the case of generating an operative field image for viewing a video of an operation afterward, that is to say, for reviewing the operation afterward for study or the like, and the like. In such a case, for example, it is considered to enhance the visibility of an anterior capsule, a posterior capsule, and a cornea without enhancing shadow contrast, by excluding the influence of the oblique illumination, for paying attention to the occurrence of capsule rupture being a complication, and the like.

Hereinafter, it is assumed that there is a plurality of modes including an operative mode and an observation mode, as a combining mode to be set when the mix processing or the like is performed and an operative field image is generated.

Here, the operative mode is a mode for generating an operative field image to be presented to an operator who actually performs an operation, and the observation mode is a mode for generating an operative field image to be presented to a supervisory doctor, an assistant, a learner, and the like who observe the operation during the operation or after the operation.

Furthermore, more specifically, for example, a combining mode varies depending on a process (procedure) of an operation even though the use application of an operative field image, that is to say, a presentation destination of the operative field image remains the same.

If a combining mode varies for each process (procedure) of an operation even though the use application of an operative field image remains the same, the mix processing, the edge enhancement processing, and the like that vary for each of these processes can be performed. Specifically, for example, in a cataract operation, a mixing ratio, and the intensity and the presence or absence of the edge enhancement processing may be changed for each process of anterior capsulotomy, phacoemulsification, intraocular lens insertion, and the like.

Note that, hereinafter, the description will be given of an example in which a combining mode is defined for each combination of a presentation destination (use application) of an operative field image and a process of an operation, but a combining mode may be defined only for at least either one of a presentation destination of an operative field image or a process of an operation.

Here, operative field image generation processing to be performed by the microscope system 101 when an operative field image is to be generated will be described with reference to a flowchart in FIG. 13.

In step S11, in accordance with the control of the microscope control unit 124, the image acquisition unit 121 acquires a coaxial illumination image and an oblique illumination image and supplies the acquired images to the image processing unit 123.

For example, in a case where the image acquisition unit 121 has the configuration illustrated in FIG. 7, the microscope control unit 124 controls the infrared light source 151 and the visible light source 152 to simultaneously emit the oblique illumination light and the coaxial illumination light to an operation target eye. In other words, the control is performed in such a manner that the coaxial illumination and the oblique illumination are simultaneously performed.

Then, the shooting unit 155 obtains an oblique illumination image and a coaxial illumination image by optically separating the oblique illumination light and the coaxial illumination light that have entered from the operation target eye, or simultaneously receiving and photoelectrically converting the oblique illumination light and the coaxial illumination light.

In addition, for example, in a case where the image acquisition unit 121 has the configuration illustrated in FIG. 12, the microscope control unit 124 controls the visible light source 311 and the visible light source 152 to alternately emit the oblique illumination light and the coaxial illumination light to the operation target eye. In other words, the control is performed in such a manner that the coaxial illumination and the oblique illumination are alternately performed.

Then, the shooting unit 312 obtains an oblique illumination image and a coaxial illumination image in a time-shared manner by receiving and photoelectrically converting the oblique illumination light or the coaxial illumination light that has entered from the operation target eye.

In step S12, the image processing unit 123 selects a combining mode. For example, the image processing unit 123 selects a combining mode on the basis of a signal supplied from an input manipulation unit (not illustrated) in accordance with an input manipulation of an operator or the like.

Aside from this, for example, the display unit 112 including a turned-on power source provided inside the display unit 112 to which the image processing unit 123 is connected may be identified, and a presentation destination of an operative field image may be identified on the basis of the identification result. In this case, for example, a combining mode is selected on the basis of a presentation destination of an operative field image that is predefined for the display unit 112 including a turned-on power source, and a process of an operation that has been identified by a method of some sort.

For example, a process of an operation may be identified by the image processing unit 123 performing image recognition or the like on the coaxial illumination image and the oblique illumination image that have been supplied from the image acquisition unit 121, or may be identified on the basis of a manipulation input performed by an operator or the like.

Note that only one combining mode may be selected or a plurality of combining modes may be selected. Hereinafter, the description will be given assuming that only one combining mode is selected, but in a case where a plurality of combining modes is selected, processes in steps S13 to S15, which will be described below, are performed for each of the combining modes.

In step S13, on the basis of the combining mode selected in step S12, the image processing unit 123 performs the edge enhancement processing as necessary on the coaxial illumination image and the oblique illumination image that have been supplied from the image acquisition unit 121.

Here, for example, whether to perform the edge enhancement processing is decided on the basis of the combining mode for each of the coaxial illumination image and the oblique illumination image. Specifically, for example, in a case where the observation mode is selected as a combining mode, the edge enhancement processing is performed on the coaxial illumination image and the edge enhancement processing is not performed on the oblique illumination image.

Note that, in performing the edge enhancement processing, for example, object recognition or the like may be performed on the coaxial illumination image and the oblique illumination image, an operative tool, a specific portion of an eye, and the like may be detected, and the edge enhancement processing may be performed for each region in accordance with the detection result. In other words, the edge enhancement processing may be performed only on a specific region of an image, or an enhancement degree of the edge enhancement processing may be decided for each region of an image.

In step S14, on the basis of the combining mode selected in step S12, the image processing unit 123 decides mixing ratios of the coaxial illumination image and the oblique illumination image.

For example, the image processing unit 123 decides a mixing ratio predefined for the combining mode, as a mixing ratio to be used in the mix processing.

In addition, for example, an operative tool or a specific portion of an eye may be detected from the coaxial illumination image and the oblique illumination image by the image processing unit 123 performing object recognition or the like, and a mixing ratio may be decided for each region in accordance with the detection result. For example, an operative mode in which a process of an operation is phacoemulsification is assumed to be selected as a combining mode. In this case, for example, the image processing unit 123 decides mixing ratios in such a manner that a mixing ratio of the oblique illumination image becomes higher than a mixing ratio of the coaxial illumination image, and a mixing ratio of a region in the oblique illumination image that includes the operative tool becomes particularly higher than other regions.

Note that the processes in steps S13 and S14 may be simultaneously performed.

In step S15, the image processing unit 123 mixes the coaxial illumination image and the oblique illumination image that have been subjected to the edge enhancement processing as necessary in step S13, at the mixing ratios decided in step S14, and generates an operative field image. Note that, in a case where the oblique illumination image is an infrared light image, as described above, the oblique illumination image and a Y image of the coaxial illumination image are mixed (combined), and an operative field image is generated.

When an operative field image is obtained, the image processing unit 123 supplies the obtained operative field image to the display unit 112 and causes the obtained operative field image to be displayed thereon, or supplies the obtained operative field image to the image recording unit 122 and causes the obtained operative field image to be recorded therein, and the operative field image generation processing ends.

In the above-described manner, the microscope system 101 acquires a coaxial illumination image and an oblique illumination image and generates an operative field image. With this configuration, an operator or the like can easily obtain an operative field image appropriate for each combining mode, that is to say, an operative field image with high visibility that corresponds to a use application, without performing a troublesome manipulation such as the adjustment of an illumination intensity. Accordingly, the operator can comfortably perform an operation in a good operative field.

<Description of specular reflection component removing processing>

In addition, processing of removing a specular reflection component in an operative field image can also be performed as image processing in the image processing unit 123.

In an ophthalmic microscope, because of the use of the coaxial illumination, a specular reflection component is generally present at the center part of an operative field. This has led to the visibility deterioration of the operative field also in an ophthalmic optical microscope, and even in the case of an ophthalmic video microscope, information regarding the operative field can disappear by the occurrence of clipped highlights.

In the microscope system 101, in a coaxial illumination image, only specular reflection of the coaxial illumination light exists as illustrated in FIG. 14, for example, and in an oblique illumination image, only specular reflection of the oblique illumination light exists as illustrated in FIG. 15, for example. In addition, in an operative field image, specular reflections of the coaxial illumination light and the oblique illumination light exist as illustrated in FIG. 16, for example. Note that, in FIGS. 14 to 16, a portion corresponding to that in FIG. 4 is denoted with the same reference numeral, and the description thereof will be appropriately omitted.

For example, FIG. 14 illustrates a coaxial illumination image. In this example, in a portion indicated by an arrow Q51 at the approximate center of the coaxial illumination image, a region with clipped highlights or a luminance close to clipped highlights is generated by the coaxial illumination light being specularly-reflected on a corneal surface or the like, and visibility in the region deteriorates. Hereinafter, a region in which clipped highlights are generated or which has a luminance close to clipped highlights due to the specular reflection will also be specifically referred to as a specular reflection region.

In a similar manner, FIG. 15 illustrates an oblique illumination image, and in this example, in a portion indicated by an arrow Q61 at the approximate center of the oblique illumination image, a specular reflection region with clipped highlights or a luminance close to clipped highlights is generated by the oblique illumination light being specularly-reflected on a corneal surface or the like, and visibility in the specular reflection region deteriorates.

Accordingly, even if an operative field image is generated by simply mixing the coaxial illumination image and the oblique illumination image, as illustrated in FIG. 16, for example, the influence of the specular reflections of the coaxial illumination light and the oblique illumination light, that is to say, a specular reflection component remains. FIG. 16 illustrates an operative field image, and in this example, in a portion indicated by an arrow Q71 at the approximate center of the operative field image, specular reflection regions are generated by the coaxial illumination light and oblique illumination light being specularly-reflected on the corneal surface or the like, and visibilities in these specular reflection region deteriorate.

Nevertheless, in the coaxial illumination image, only the specular reflection region of the coaxial illumination light exists, and the specular reflection region of the oblique illumination light does not exist. In a similar manner, in the oblique illumination image, only the specular reflection region of the oblique illumination light exists, and the specular reflection region of the coaxial illumination light does not exist.

Accordingly, in the image processing unit 123, if image information of the coaxial illumination image is used, the influence of the specular reflection in the specular reflection region of the oblique illumination image can be removed. Conversely, if image information of the oblique illumination image is used, the influence of the specular reflection in the specular reflection region of the coaxial illumination image can be removed. From these points, in the image processing unit 123, an operative field image in which the influence of the specular reflection has been reduced, that is to say, a component of the specular reflection has been removed can be obtained.

Specifically, for example, only in one image of the coaxial illumination image and the oblique illumination image, a region having a luminance value of clipped highlights or a luminance value close to clipped highlights, that is to say, a region having a luminance value being equal to or larger than a threshold value is regarded as a specular reflection region.

When a specular reflection region is detected from the coaxial illumination image and the oblique illumination image, after that, a mixing ratio is decided for each region in accordance with the detection result of the specular reflection region, and an operative field image is generated by the mix processing.

For example, the coaxial illumination image and the oblique illumination image are both assumed to be visible light images.

In this case, for example, as for a portion in the coaxial illumination image that includes a specular reflection region, a mixing ratio of the coaxial illumination image is set to about 0, and an operative field image is generated by substantially using only the oblique illumination image. In a similar manner, for example, as for a portion in the oblique illumination image that includes a specular reflection region, a mixing ratio of the oblique illumination image is set to about 0, and an operative field image is generated by substantially using only the coaxial illumination image. With this configuration, an operative field image not including a specular reflection region can be obtained.

In addition, for example, a coaxial illumination image is assumed to be a visible light image and an oblique illumination image is assumed to be an infrared light image.

In this case, for example, as for a portion in the coaxial illumination image that includes a specular reflection region, a mixing ratio of a Y image of the coaxial illumination image is set to about 0, and a mixed luminance image is generated by substantially using only the oblique illumination image. In a similar manner, for example, as for a portion in the oblique illumination image that includes a specular reflection region, a mixing ratio of the oblique illumination image is set to using 0, and a mixed luminance image is generated by substantially using only the Y image of the coaxial illumination image. Then, an operative field image is generated from the obtained mixed luminance image, and a Cb image and a Cr image of the coaxial illumination image. With this configuration, an operative field image not including a specular reflection region can be obtained.

In addition, in a case where a specular reflection region is detected, image processing of suppressing the occurrence of clipped highlights by lowering a digital gain only for the specular reflection region can also be performed.

For example, in the image processing unit 123, in a case where gain adjustment is performed by multiplying a coaxial illumination image and an oblique illumination image by a digital gain, and then, the mix processing or the like is performed, the occurrence of clipped highlights can be suppressed by lowering a digital gain of a specular reflection region of the coaxial illumination image and the oblique illumination image.

Furthermore, for example, the microscope control unit 124 may perform exposure control such as control of shortening an exposure time of a pixel column (line) corresponding to the specular reflection region, on the basis of the detection result of the specular reflection region that has been obtained by the image processing unit 123.

Here, specular reflection component removing processing to be performed by the microscope system 101 when an operative field image in which specular reflection components, that is to say, clipped highlights are reduced is to be generated will be described with reference to a flowchart in FIG. 17. Note that, because a process in step S41 is similar to the process in step S11 of FIG. 13, the description thereof will be omitted.

In step S42, on the basis of the coaxial illumination image and the oblique illumination image that have been supplied from the image acquisition unit 121, the image processing unit 123 detects a specular reflection region from the coaxial illumination image.

More specifically, the image processing unit 123 compares the coaxial illumination image and the oblique illumination image for each pixel, and detects, as a specular reflection region, a region corresponding to a pixel in which a luminance value of a pixel in the coaxial illumination image is equal to or larger than a threshold value, but a luminance value of a pixel in the oblique illumination image that is in the same positional relationship as the pixel is less than the threshold value.

In step S43, on the basis of the coaxial illumination image and the oblique illumination image that have been supplied from the image acquisition unit 121, the image processing unit 123 detects a specular reflection region from the oblique illumination image.

More specifically, the image processing unit 123 compares the coaxial illumination image and the oblique illumination image for each pixel, and detects, as a specular reflection region, a region corresponding to a pixel in which a luminance value of a pixel in the oblique illumination image is equal to or larger than a threshold value, but a luminance value of a pixel in the coaxial illumination image that is in the same positional relationship as the pixel is less than the threshold value.

Note that, more specifically, the processes in steps S42 and S43 are simultaneously performed. In addition, a detection method of a specular reflection region may be any method. For example, a region in which a difference value of luminance values of pixels in the same positional relationship of the coaxial illumination image and the oblique illumination image becomes a threshold value or more may be detected as a specular reflection region.

In step S44, on the basis of the detection result of the specular reflection regions of the coaxial illumination image and the oblique illumination image, the image processing unit 123 generates an operative field image in which specular reflection components are reduced.

More specifically, for example, as described above, the image processing unit 123 decides a mixing ratio for each region in such a manner that almost only the oblique illumination image is used for a region in the coaxial illumination image that corresponds to the specular reflection region, and almost only the coaxial illumination image is used for a region in the oblique illumination image that corresponds to the specular reflection region. Then, the image processing unit 123 mixes the coaxial illumination image and the oblique illumination image on the basis of the decided mixing ratio, and generates an operative field image in which specular reflection components are reduced (removed).

Note that, in the mix processing, as described with reference to FIG. 13, an operative field image may be generated considering a combining mode, an object recognition result, and the like as well. In addition, the edge enhancement processing may be appropriately performed on the coaxial illumination image and the oblique illumination image.

When an operative field image is obtained, the image processing unit 123 supplies the obtained operative field image to the display unit 112 and causes the obtained operative field image to be displayed thereon, or supplies the obtained operative field image to the image recording unit 122 and causes the obtained operative field image to be recorded thereon, and the specular reflection component removing processing ends.

In the above-described manner, the microscope system 101 acquires a coaxial illumination image and an oblique illumination image, detects a specular reflection region, and generates an operative field image on the basis of the detection result. With this configuration, an operative field image which includes less specular reflection and has high visibility can be obtained. As a result, an operator can comfortably perform an operation in a good operative field.

<Description of pupil region detection processing>

Furthermore, in the image processing unit 123, a portion of an eye can be detected highly accurately using a coaxial illumination image and an oblique illumination image that have been obtained using mutually different illumination methods, that is to say, the coaxial illumination and the oblique illumination. Hereinafter, the description will be given of an example in which a pupil region is detected as a specific portion of an eye.

In the coaxial illumination in which transillumination can be obtained, the inside of a pupil is observed to be bright as in the example illustrated in FIG. 3, for example, whereas in the oblique illumination in which transillumination cannot be obtained, the inside of a pupil is observed to be darker than the surrounding as in the example illustrated in FIG. 2, for example. Accordingly, a region of the pupil is bright on the coaxial illumination image and the region of the pupil is dark on the oblique illumination image.

In the image processing unit 123, by using such a property, a pupil region can be detected easily and highly accurately by comparing the coaxial illumination image and the oblique illumination image.

Specifically, for example, when a position represented by a two-dimensional xy coordinate system is denoted by (x, y), a luminance value of a pixel located at the position (x, y) on the coaxial illumination image is denoted by YP (x, y), and a luminance value of a pixel located at the position (x, y) on the oblique illumination image is denoted by YS (x, y).

In addition, a coefficient for correcting a difference in sensitivity of the coaxial illumination image is denoted by $\alpha$, and a coefficient for correcting a difference in sensitivity of the oblique illumination image is denoted by $\beta$. Furthermore, when a luminance difference between the coaxial illumination image and the oblique illumination image at the position (x, y) is denoted by I (x, y), the luminance difference I (x, y) can be obtained by the following formula (1).

$$I (x, y) = \alpha \times YP (x, y) - \beta \times YS (x, y) \dots (1)$$

As described above, because a pupil region is bright on the coaxial illumination image and the pupil region is dark on the oblique illumination image, in the pupil region, the luminance difference I (x, y) becomes a sufficiently large value as compared with a region provided on the outside of the pupil. Thus, for example, by performing threshold value processing of the luminance difference I (x, y), a pupil region can be easily detected. In this case, a region in which the luminance difference I (x, y) becomes equal to or larger than a predefined threshold value is regarded as a pupil region.

For example, in a cataract operation, a region provided on the inside of a pupil is a center region of an operative field, and brightness of the operative field varies in accordance with the progress (process) of the operation.

Thus, for example, in the cataract operation, when a pupil region is detected by the image processing unit 123, shooting control can be performed in the following manner. For example, exposure control suitable for a portion of the detected pupil region can be performed, and a shooting position can be controlled in such a manner that the pupil region always comes to the center of an operative field image. By performing the shooting control in this manner, an operator can obtain an operative field with appropriate brightness, can observe an operative field image in which the pupil region is located at an image center, and can comfortably perform an operation.

In addition, aside from these, for example, in a process of intraocular lens insertion in a cataract operation, guide information indicating a lens direction or the like may be displayed together with the rim of the pupil region, with being superimposed on the pupil region of the operative field image, and a detection result of the pupil region may be used for tracking processing for identifying the lens direction with respect to an eyeball, or the like.

Here, the pupil region detection processing to be performed by the microscope system 101 when a pupil region is to be detected will be described with reference to the flowchart in FIG. 18. Note that, because a process in step S71 is similar to the process in step S11 of FIG. 13, the description thereof will be omitted.

In step S72, the image processing unit 123 performs detection processing of a pupil region on the basis of the coaxial illumination image and the oblique illumination image that have been supplied from the image acquisition unit 121.

More specifically, the image processing unit 123 detects a pupil region by calculating the above-described formula (1), and comparing the resultant luminance difference I (x, y) with a threshold value.

In step S73, the image processing unit 123 determines whether or not a pupil region has been detected as a result of the detection processing in step S72.

In a case where it is determined in step S73 that a pupil region has not been detected, after that, the processing returns to step S71, and the above-described processes are repeatedly performed.

In contrast to this, in a case where it is determined in step S73 that a pupil region has been detected, the image processing unit 123 supplies the detection result of the pupil region to the microscope control unit 124, and the processing proceeds to step S74.

In step S74, the microscope control unit 124 performs control corresponding to the detection result of the pupil region that has been supplied from the image processing unit 123.

For example, the microscope control unit 124 performs exposure control for the shooting of a coaxial illumination image and an oblique illumination image that is performed by the image acquisition unit 121, in such a manner that a pupil region in the coaxial illumination image and the oblique illumination image has appropriate brightness. More specifically, control of an exposure time in the shooting, and the like are performed.

In addition, for example, the microscope control unit 124 controls a shooting position (shooting direction) to be used by the image acquisition unit 121, in such a manner that a pupil region is located at the position of the image center in the coaxial illumination image and the oblique illumination image, by physically inclining or translating an optical system.

Aside from these, for example, the microscope control unit 124 may cause a coaxial illumination image and an oblique illumination image to be generated, by controlling the image acquisition unit 121 to perform trimming of a shot image in such a manner that a pupil region is located at the position of an image center in the coaxial illumination image and the oblique illumination image.

Furthermore, in accordance with a detection result of a pupil region, the image processing unit 123 may perform image processing of generating, as guide information, an image indicating a rim portion (boundary portion) of the pupil region, and an image indicating a direction of an intraocular lens at a pupil region position, and superimposing the guide information onto an operative field image. In this case, the image processing unit 123 supplies the operative field image on which the guide information is superimposed, to the display unit 112, and causes the operative field image to be displayed thereon. Aside from this, the image processing unit 123 may perform tracking processing of a specific portion of an eye such as a pupil region, on the basis of a detection result of a pupil region.

In step S75, the image processing unit 123 determines whether or not to end the processing. In a case where it is determined in step S75 that the processing is not to be ended yet, the processing returns to step S71, and the above-described processes are repeatedly performed.

In contrast to this, in a case where it is determined in step S75 that the processing is to be ended, the pupil region detection processing ends.

In the above-described manner, the microscope system 101 acquires a coaxial illumination image and an oblique illumination image and detects a pupil region, and performs control such as shooting control on the basis of the detection result. With this configuration, for example, an operative field image with appropriate brightness can be obtained, a pupil can be observed at the center of an operative field, and an operator can comfortably perform an operation in a good operative field.

Note that, here, the description has been given of an example in which a pupil region is detected as a specific portion of an eye, but any other portions may be detected from a coaxial illumination image and an oblique illumination image.

<Information acquisition and information presentation that utilize property of wavelength>

Furthermore, for example, in a case where the image acquisition unit 121 has the configuration illustrated in FIG. 7, image processing that uses information obtained from an infrared light image can also be performed.

More specifically, for example, when an image is shot using light having a wavelength different from that of visible light, such as infrared light, information that cannot be obtained from a visible light image can be obtained from the shot image. Thus, the information obtained in this manner can be used for image processing.

Specifically, for example, according to "the abstracting journal of the 27th meeting of Japan Glaucoma Society: Four Examples of Positioning of Sclera Flaps Using Infrared Ray Imaging before Filtration Bleb Needling Revisions", it has been reported that, when shooting is performed using infrared light, the visibility of an outflow pathway of aqueous humor that has been created in a trabeculectomy operation of glaucoma, and is located on a white part of an eye is enhanced.

In the microscope system 101, if the image acquisition unit 121 has the configuration illustrated in FIG. 7, for example, an oblique illumination image being an infrared light image can be obtained. Thus, if an operative field image is generated by mixing the oblique illumination image and a coaxial illumination image, the visibility of a sclera flap portion provided with an outflow pathway of aqueous humor can be enhanced, and a better operative field can be obtained. Accordingly, by performing an operation while viewing such an operative field image, an operator can perform the operation more comfortably.

Note that, in this case, in the image processing unit 123, for example, the sclera flap portion and an outflow pathway portion of aqueous humor may be detected from the oblique illumination image, and a mixing ratio may be decided for each region in such a manner that a mixing ratio of the oblique illumination image becomes higher in a region of the sclera flap portion and the outflow pathway portion of aqueous humor.

In addition, in the image processing unit 123, image processing of generating an operative field image on which guide information is superimposed, by detecting a rim of the sclera flap from the oblique illumination image being an infrared light image, and superimposing information indicating the detected rim portion onto the coaxial illumination image can also be performed. With this configuration, appropriate guide information can be presented without dyeing an eyeball of a patient, and an operator can perform an operation more comfortably.

As described above, according to the microscope system 101, by separating acquiring a coaxial illumination image and an oblique illumination image, a good operative field can be easily obtained by subsequent image processing, more appropriate shooting control can be performed, and it becomes possible for an operator to comfortably perform an operation. For example, according to the microscope system 101, the following advantages (1) to (4), and the like can be obtained.

Advantage (1): removal of specular reflection component

For example, because disturbing specular reflection becomes inexistent at the center of an operative field if the specular reflection component removing processing described with reference to FIG. 17 is performed, the visibility is enhanced, and it becomes possible to concentrate on a surgery.

Advantage (2): generation of operative field image having influence degree of illumination varied for each region

For example, if the operative field image generation processing described with reference to FIG. 13 is performed, an operative field image having both of three-dimensionality caused by shadow contrast and contrast caused by transillumination can be obtained, and a surgery is facilitated. In addition, by appropriately deciding a mixing ratio in the mix processing, it becomes possible to obtain an operative field image in which information important for each observer is enhanced.

Advantage (3): detection of pupil region

For example, if the pupil region detection processing described with reference to FIG. 18 is performed, a pupil region can be easily detected, and in addition, by performing exposure control, control of a shooting position, and the like using the detection result, a good operative field can be obtained.

Advantage (4): information acquisition using specific wavelength component

For example, as in the above-described example of visibility enhancement of an outflow pathway of aqueous humor, by shooting an image of a wavelength different from that of visible light, such as infrared light, information useful for an operation that cannot be obtained from a visible light image can be obtained. By appropriately superimpose-displaying the information obtained in this manner, onto an operative field image, it becomes possible for an operator to perform an operation more comfortably.

<Example of computer configuration>

Incidentally, the above-described series of processes may be performed by hardware or may be performed by software. When the series of processes are performed by software, a program forming the software is installed into a computer. Examples of the computer include a computer that is incorporated in dedicated hardware and a general-purpose computer that can perform various types of function by installing various types of program.

FIG. 19 is a block diagram illustrating a configuration example of the hardware of a computer that performs the above-described series of processes with a program.

In the computer, a central processing unit (CPU) 501, read only memory (ROM) 502, and random access memory (RAM) 503 are mutually connected by a bus 504.

Further, an input/output interface 505 is connected to the bus 504. Connected to the input/output interface 505 are an input unit 506, an output unit 507, a recording unit 508, a communication unit 509, and a drive 510.

The input unit 506 includes a keyboard, a mouse, a microphone, an image sensor, and the like. The output unit 507 includes a display, a speaker, and the like. The recording unit 508 includes a hard disk, a non-volatile memory, and the like. The communication unit 509 includes a network interface, and the like. The drive 510 drives a removable recording medium 511 such as a magnetic disk, an optical disc, a magneto-optical disk, and a semiconductor memory.

In the computer configured as described above, the CPU 501 loads a program that is recorded, for example, in the recording unit 508 onto the RAM 503 via the input/output interface 505 and the bus 504, and executes the program, thereby performing the above-described series of processes.

For example, programs to be executed by the computer (CPU 501) can be recorded and provided in the removable recording medium 511, which is a packaged medium or the like. In addition, programs can be provided via a wired or wireless transmission medium such as a local area network, the Internet, and digital satellite broadcasting.

In the computer, by mounting the removable recording medium 511 onto the drive 510, programs can be installed into the recording unit 508 via the input/output interface 505. Programs can also be received by the communication unit 509 via a wired or wireless transmission medium, and installed into the recording unit 508. In addition, programs can be installed in advance into the ROM 502 or the recording unit 508.

Note that a program executed by the computer may be a program in which processes are chronologically carried out in a time series in the order described herein or may be a program in which processes are carried out in parallel or at necessary timing, such as when the processes are called.

In addition, embodiments of the present disclosure are not limited to the above-described embodiments, and various alterations may occur insofar as they are within the scope of the present disclosure.

For example, the present technology can adopt a configuration of cloud computing, in which a plurality of devices shares a single function via a network and perform processes in collaboration.

Furthermore, each step in the above-described flowcharts can be executed by a single device or shared and executed by a plurality of devices.

In addition, when a single step includes a plurality of processes, the plurality of processes included in the single step can be executed by a single device or shared and executed by a plurality of devices.

The advantageous effects described herein are not limited, but merely examples. Any other advantageous effects may also be attained.

Additionally, the present technology may also be configured as below.

(1) An image processing device including:

an image acquisition unit configured to acquire a coaxial illumination image obtained by performing coaxial illumination on an eye being an operation target, and an oblique illumination image obtained by performing oblique illumination on the eye; and
an image processing unit configured to generate an operative field image of the eye on the basis of the coaxial illumination image and the oblique illumination image, or detect a specific region on the basis of at least either one of the coaxial illumination image or the oblique illumination image.

(2) The image processing device according to (1), in which coaxial illumination light of the coaxial illumination and oblique illumination light of the oblique illumination are rays of light that have mutually different wavelengths, and
the image processing device further includes a control unit configured to control the coaxial illumination and the oblique illumination to be simultaneously performed.

(3) The image processing device according to (2), in which the image acquisition unit acquires the coaxial illumination image and the oblique illumination image by optically separating the coaxial illumination light and the oblique illumination light.

(4) The image processing device according to (2), in which the image acquisition unit acquires the coaxial illumination image and the oblique illumination image from data obtained by receiving the coaxial illumination light and the oblique illumination light.

(5) The image processing device according to (1), further including a control unit configured to control the coaxial illumination and the oblique illumination to be alternately performed,
in which the image acquisition unit acquires the coaxial illumination image and the oblique illumination image in a time-shared manner.

(6) The image processing device according to any one of (1) to (5), in which the image processing unit generates the operative field image by mixing the coaxial illumination image and the oblique illumination image at a predetermined mixing ratio.

(7) The image processing device according to (6), in which the image processing unit decides the mixing ratio for each region.

(8) The image processing device according to (7), in which the image processing unit detects a region of an operative tool or a region of a specific portion of the eye on the basis of at least either one of the coaxial illumination image or the oblique illumination image, and decides the mixing ratio for each region on the basis of the detection result.

(9) The image processing device according to any one of (6) to (8), in which the image processing unit decides the mixing ratio on the basis of at least either one of a use application of the operative field image or a process of an operation of the eye.

(10) The image processing device according to any one of (6) to (9), in which the image processing unit generates the operative field image after performing enhancement processing on at least either one of the coaxial illumination image or the oblique illumination image.

(11) The image processing device according to (10), in which the image processing unit decides whether to perform the enhancement processing, on the basis of at least either one of a use application of the operative field image or a process of an operation of the eye, for each of the coaxial illumination image and the oblique illumination image.

(12) The image processing device according to any one of (1) to (11), in which the image processing unit detects a specular reflection region on the basis of the coaxial illumination image and the oblique illumination image, and generates the operative field image in which a specular reflection component is reduced, on the basis of the detection result.

(13) The image processing device according to any one of (1) to (12), in which the image processing unit detects a region of a specific portion of the eye as the specific region.

(14) The image processing device according to (13), in which the image processing unit compares the coaxial illumination image and the oblique illumination image, and detects a pupil region of the eye.

(15) The image processing device according to (13) or (14), further including a control unit configured to perform shooting control of the coaxial illumination image and the oblique illumination image on the basis of a detection result of the region of the specific portion.

(16) The image processing device according to (15), in which the control unit performs exposure control or control of a shooting position as the shooting control.

(17) The image processing device according to any one of (13) to (16), in which the image processing unit generates guide information for aiding an operation of the eye, on the basis of a detection result of the region of the specific portion of the eye.

(18) An image processing method including the steps of:

acquiring a coaxial illumination image obtained by performing coaxial illumination on an eye being an operation target, and an oblique illumination image obtained by performing oblique illumination on the eye; and
generating an operative field image of the eye on the basis of the coaxial illumination image and the oblique illumination image, or detecting a specific region on the basis of at least either one of the coaxial illumination image or the oblique illumination image.

(19) An operating microscope system including:

a coaxial illumination light source configured to perform coaxial illumination by emitting coaxial illumination light to an eye being an operation target;
an oblique illumination light source configured to perform oblique illumination by emitting oblique illumination light to the eye;
an image acquisition unit configured to acquire a coaxial illumination image obtained by performing the coaxial illumination, and an oblique illumination image obtained by performing the oblique illumination; and
an image processing unit configured to generate an operative field image of the eye on the basis of the coaxial illumination image and the oblique illumination image, or detect a specific region on the basis of at least either one of the coaxial illumination image or the oblique illumination image.

Reference Signs List

| 101 | microscope system |
| 111 | ophthalmic video microscope |
| 121 | image acquisition unit |
| 122 | image recording unit |
| 123 | image processing unit |
| 124 | microscope control unit |

**Claims**

1. An image processing device comprising:

an image acquisition unit configured to acquire a coaxial illumination image obtained by performing coaxial illumination on an eye being an operation target, and an oblique illumination image obtained by performing oblique illumination on the eye; and
an image processing unit configured to generate an operative field image of the eye on the basis of the coaxial illumination image and the oblique illumination image, or detect a specific region on the basis of at least either one of the coaxial illumination image or the oblique illumination image.

2. The image processing device according to claim 1, wherein coaxial illumination light of the coaxial illumination and oblique illumination light of the oblique illumination are rays of light that have mutually different wavelengths, and
the image processing device further includes a control unit configured to control the coaxial illumination and the oblique illumination to be simultaneously performed.

3. The image processing device according to claim 2, wherein the image acquisition unit acquires the coaxial illumination image and the oblique illumination image by optically separating the coaxial illumination light and the oblique illumination light.

4. The image processing device according to claim 2, wherein the image acquisition unit acquires the coaxial illumination image and the oblique illumination image from data obtained by receiving the coaxial illumination light and the oblique illumination light.

5. The image processing device according to claim 1, further comprising a control unit configured to control the coaxial illumination and the oblique illumination to be alternately performed,
wherein the image acquisition unit acquires the coaxial illumination image and the oblique illumination image in a time-shared manner.

6. The image processing device according to claim 1, wherein the image processing unit generates the operative field image by mixing the coaxial illumination image and the oblique illumination image at a predetermined mixing ratio.

7. The image processing device according to claim 6, wherein the image processing unit decides the mixing ratio for each region.

8. The image processing device according to claim 7, wherein the image processing unit detects a region of an operative tool or a region of a specific portion of the eye on the basis of at least either one of the coaxial illumination image or the oblique illumination image, and decides the mixing ratio for each region on the basis of the detection result.

9. The image processing device according to claim 6, wherein the image processing unit decides the mixing ratio on the basis of at least either one of a use application of the operative field image or a process of an operation of the eye.

10. The image processing device according to claim 6, wherein the image processing unit generates the operative field image after performing enhancement processing on at least either one of the coaxial illumination image or the oblique illumination image.

11. The image processing device according to claim 10, wherein the image processing unit decides whether to perform the enhancement processing, on the basis of at least either one of a use application of the operative field image or a process of an operation of the eye, for each of the coaxial illumination image and the oblique illumination image.

12. The image processing device according to claim 1, wherein the image processing unit detects a specular reflection region on the basis of the coaxial illumination image and the oblique illumination image, and generates the operative field image in which a specular reflection component is reduced, on the basis of the detection result.

13. The image processing device according to claim 1, wherein the image processing unit detects a region of a specific portion of the eye as the specific region.

14. The image processing device according to claim 13, wherein the image processing unit compares the coaxial illumination image and the oblique illumination image, and detects a pupil region of the eye.

15. The image processing device according to claim 13, further comprising a control unit configured to perform shooting control of the coaxial illumination image and the oblique illumination image on the basis of a detection result of the region of the specific portion.

16. The image processing device according to claim 15, wherein the control unit performs exposure control or control of a shooting position as the shooting control.

17. The image processing device according to claim 13, wherein the image processing unit generates guide information for aiding an operation of the eye, on the basis of a detection result of the region of the specific portion of the eye.

18. An image processing method comprising the steps of:

acquiring a coaxial illumination image obtained by performing coaxial illumination on an eye being an operation target, and an oblique illumination image obtained by performing oblique illumination on the eye; and
generating an operative field image of the eye on the basis of the coaxial illumination image and the oblique illumination image, or detecting a specific region on the basis of at least either one of the coaxial illumination image or the oblique illumination image.

19. An operating microscope system comprising:

a coaxial illumination light source configured to perform coaxial illumination by emitting coaxial illumination light to an eye being an operation target;

an oblique illumination light source configured to perform oblique illumination by emitting oblique illumination light to the eye;

an image acquisition unit configured to acquire a coaxial illumination image obtained by performing the coaxial illumination, and an oblique illumination image obtained by performing the oblique illumination; and

an image processing unit configured to generate an operative field image of the eye on the basis of the coaxial illumination image and the oblique illumination image, or detect a specific region on the basis of at least either one of the coaxial illumination image or the oblique illumination image.

# FIG. 1

## FIG. 2

41

**FIG. 3**

# FIG. 4

# FIG. 5

## FIG. 6

OPHTHALMIC VIDEO MICROSCOPE

111

122

IMAGE
RECORDING
UNIT

121

IMAGE
ACQUISITION
UNIT

123

IMAGE
PROCESSING
UNIT

124

MICROSCOPE
CONTROL
UNIT

DISPLAY
UNIT ~112-1

DISPLAY
UNIT ~112-2

101

## FIG. 7

COAXIAL
ILLUMINATION
IMAGE

155

IMAGE
PROCESSING
UNIT

~123

154

OBLIQUE
ILLUMINATION
IMAGE

151

153  152

~121

~E11

# FIG. 8

# FIG.9

# FIG. 10

# FIG. 11

155

SHOOTING UNIT

242

241

283

251

243

281          282

**FIG. 12**

COAXIAL
ILLUMINATION
IMAGE

312

IMAGE
PROCESSING ~123
UNIT

154

OBLIQUE
ILLUMINATION
IMAGE

311

152

121

E11

# FIG. 13

```
┌─────────────────────────────────────┐
│   START OF OPERATIVE FIELD IMAGE     │
│      GENERATION PROCESSING           │
└─────────────────────────────────────┘
                  │
                  ▼
┌─────────────────────────────────────┐ S11
│   ACQUIRE COAXIAL ILLUMINATION IMAGE │
│    AND OBLIQUE ILLUMINATION IMAGE    │
└─────────────────────────────────────┘
                  │
                  ▼
┌─────────────────────────────────────┐ S12
│         SELECT COMBINING MODE        │
└─────────────────────────────────────┘
                  │
                  ▼
┌─────────────────────────────────────┐ S13
│  PERFORM EDGE ENHANCEMENT PROCESSING │
│     ON BASIS OF COMBINING MODE       │
└─────────────────────────────────────┘
                  │
                  ▼
┌─────────────────────────────────────┐ S14
│     DECIDE MIXING RATIO ON BASIS OF  │
│          COMBINING MODE              │
└─────────────────────────────────────┘
                  │
                  ▼
┌─────────────────────────────────────┐ S15
│ MIX COAXIAL ILLUMINATION IMAGE AND OBLIQUE │
│ ILLUMINATION IMAGE, AND GENERATE OPERATIVE │
│            FIELD IMAGE               │
└─────────────────────────────────────┘
                  │
                  ▼
             ┌──────────┐
             │   END    │
             └──────────┘
```

## FIG. 14

## FIG. 15

## FIG. 16

# FIG. 17

```
┌──────────────────────────────────────────┐
│   START OF SPECULAR REFLECTION            │
│   COMPONENT REMOVING PROCESSING           │
└──────────────────────────────────────────┘
                    │
                    ▼
┌──────────────────────────────────────────┐ S41
│   ACQUIRE COAXIAL ILLUMINATION IMAGE      │
│   AND OBLIQUE ILLUMINATION IMAGE          │
└──────────────────────────────────────────┘
                    │
                    ▼
┌──────────────────────────────────────────┐ S42
│   DETECT SPECULAR REFLECTION REGION       │
│   FROM COAXIAL ILLUMINATION IMAGE         │
└──────────────────────────────────────────┘
                    │
                    ▼
┌──────────────────────────────────────────┐ S43
│   DETECT SPECULAR REFLECTION REGION       │
│   FROM OBLIQUE ILLUMINATION IMAGE         │
└──────────────────────────────────────────┘
                    │
                    ▼
┌──────────────────────────────────────────┐ S44
│   GENERATE OPERATIVE FIELD IMAGE IN WHICH │
│   SPECULAR REFLECTION COMPONENTS ARE      │
│   REDUCED                                 │
└──────────────────────────────────────────┘
                    │
                    ▼
              ┌───────────┐
              │    END    │
              └───────────┘
```

# FIG. 18

START OF PUPIL REGION DETECTION PROCESSING

ACQUIRE COAXIAL ILLUMINATION IMAGE AND OBLIQUE ILLUMINATION IMAGE — S71

PERFORM DETECTION PROCESSING OF PUPIL REGION — S72

S73

HAS PUPIL REGION BEEN DETECTED? — NO

YES

PERFORM CONTROL CORRESPONDING TO DETECTION RESULT OF PUPIL REGION — S74

S75

END? — NO

YES

END

## FIG. 19

## INTERNATIONAL SEARCH REPORT

| International application No. |
| --- |
| PCT/JP2017/042152 |

**A. CLASSIFICATION OF SUBJECT MATTER**
Int.Cl.    G02B21/36(2006.01)i,   A61B3/13(2006.01)i,   A61F9/007(2006.01)i,
           G02B21/06(2006.01)i

According to International Patent Classification (IPC) or to both national classification and IPC

**B. FIELDS SEARCHED**

Minimum documentation searched (classification system followed by classification symbols)
Int.Cl. G02B21/36, A61B3/13, A61F9/007, G02B21/06

Documentation searched other than minimum documentation to the extent that such documents are included in the fields searched
| | |
| --- | --- |
| Published examined utility model applications of Japan | 1922–1996 |
| Published unexamined utility model applications of Japan | 1971–2018 |
| Registered utility model specifications of Japan | 1996–2018 |
| Published registered utility model applications of Japan | 1994–2018 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| Y | JP 2016-185178 A (TOPCON) 27 October 2016, claims, paragraphs [0015], [0016], [0057], [0058], [0067], | 1-11, 13, 14, 17-19 |
| A | [0103], [0104], fig. 7 & US 2016/0278635 A1, claims, paragraphs [0020], [0021], [0062], [0063], [0108], [0109] & DE 102016203487 A1 | 12, 15, 16 |
| Y | JP 2003-177327 A (LEICA MICROSYSTEMS (SCHWEIZ) AG) 27 June 2003, claims, paragraph [0018] & US 2003/0048528 | 1-11, 13, 14, 17-19 |
| A | A1, paragraphs [0033], [0034] & EP 1291696 A1 & DE 10144067 A1 | 12, 15, 16 |
| Y | JP 8-164155 A (NIKON CORP.) 25 June 1996, paragraph [0023] & US 5627613 A, column 6, lines 22-39 | 1-11, 13, 14, 17-19 |
| A | | 12, 15, 16 |

☒ Further documents are listed in the continuation of Box C.   ☐ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 16 February 2018 (16.02.2018) | 27 February 2018 (27.02.2018) |

| Name and mailing address of the ISA/ | Authorized officer |
| --- | --- |
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

**INTERNATIONAL SEARCH REPORT**

International application No.
PCT/JP2017/042152

C (Continuation). DOCUMENTS CONSIDERED TO BE RELEVANT

| Category* | Citation of document, with indication, where appropriate, of the relevant passages | Relevant to claim No. |
|---|---|---|
| Y<br><br>A | JP 2014-233469 A (NIDEK CO., LTD.) 15 December 2014, paragraph [0056] (Family: none) | 1-11, 13, 14, 17-19<br>12, 15, 16 |
| Y | JP 5-123297 A (TOPCON) 21 May 1993, claims, paragraphs [0015]-[0019], fig. 1 (Family: none) | 3 |
| Y | JP 2010-181688 A (YOKOGAWA ELECTRIC CORP.) 19 August 2010, paragraphs [0057], [0060] (Family: none) | 5 |
| Y | JP 2016-112358 A (SONY CORP.) 23 June 2016, paragraphs [0201], [0210] & US 2017/0273559 A1, paragraphs [0283], [0286] & EP 3235477 A1 | 10, 11 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2016073409 A